# FASCICULE DE BREVET EUROPEEN

(11) **EP 0 391 766 B1**
(45) Date de publication et mention de la délivrance du brevet: **24.05.1995**
(21) Numéro de dépôt: 90400815.8
(22) Date de dépôt: 23.03.1990
(51) Int. Cl.: C07D 471/18, C07D 471/08, C07D 498/18, C07D 498/08, C07D 487/18, A61K 49/00

(54) **Nouveaux ligands macrocycliques azotés, procédé de préparation, complexes métalliques formés par ces ligands, composition de diagnostic et composition thérapeutique les contenant**
Makrocyclische Stickstoffliganden Verfahren zu ihrer Herstellung, ihre Metallkomplexe, sie enthaltende Diagnostische und therapeutische Mittel
Macrocyclic nitrogen ligands, method of preparation, metal complexes formed by the ligands, diagnostic and therapeutic compositions containing them

(30) Priorité: 24.03.1989 FR 8903938
(43) Date de publication de la demande: 10.10.1990
(73) Titulaire: GUERBET S.A., F-93420 Villepinte (FR)
(72) Inventeur: Jackels, Suzan C., Winston-Salem, North Carolina 27106 (US); Meyer, Dominique, F-75013 Paris (FR)
(74) Mandataire: Le Guen, Gérard

(56) Documents cités:
- EP-A- 0 203 047
- EP-A- 0 438 206
- EP-A- 0 439 387
- WO-A-91/10645
- WO-A-91/10669
- TETRAHEDRON, vol. 37, 1981, pages 762-772, Pergamon Press, Ltd, GB; H. STETTERet al.: "Darstellung und Komplexbildung von Polyazacycloalkan-N-Essigsäuren"
- Magnetic Resonance Imaging, vol. 3, 1985, pages 11-16
- Magnetic Resonance in Medecine, vol.8(2), 1988, pages 180-190
- Houben-Weil, Georg Thieme Verlag Stuttgart New York, Band 4, Teil 1c-Reduktion I, pages 362-364
- Comprehensive Organic Chemistry, Barton D., Pergamon Press, Vol.4, page 816

## Description

La présente invention concerne de nouveaux ligands cycliques azotés et des complexes métalliques formés par ces ligands, les applications diagnostiques et thérapeutiques de ces complexes notamment en imagerie par résonance magnétique, en radiologie par rayons X, comme agents de déplacement chimique in vivo et en médecine nucléaire.

L'invention concerne également un procédé de préparation de ces ligands.

L'invention a ainsi pour objet des ligands de formule I 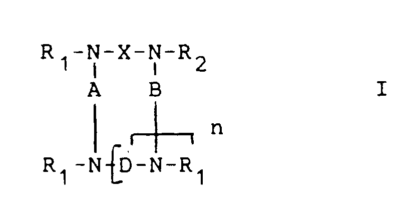
dans laquelle
A représente un groupe alkylène linéaire ou ramifié en C₂-C₅,
B représente un groupe alkylène linéaire ou ramifié en C₂-C₅,
n est égal 1,
D est choisi parmi un groupe alkylène linéaire ou ramifié en C₂-C₅ et un groupe X, X étant choisi parmi les groupes 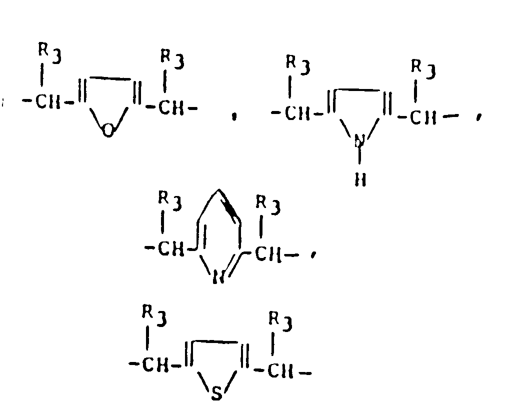
dans lesquels R₃ est choisi parmi un atome d'hydrogène et un groupe alkyle en C₁-C₃,
R₁ est choisi parmi les groupes -CH₂-COOH et
-CH₂-PO₃H₂, et
R₂ est choisi parmi un atome d'hydrogène, un groupe alkyle en C₁-C₄, hydroxyalkyle en C₂-C₄ et un groupe R₁ tel que défini précédemment.

On préfère en particulier les ligands constitués par les composés de formule I dans laquelle A et B sont identiques et choisis parmi les groupes éthylène et n-propylène, n est égal à 1, X et D sont identiques et choisis parmi les groupes 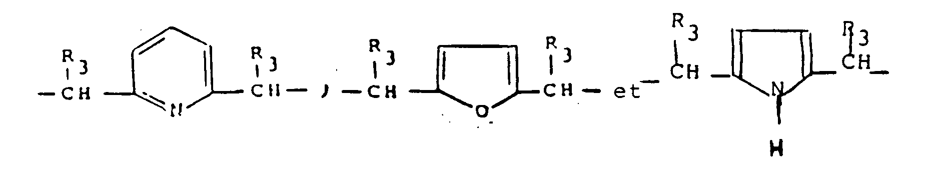
R₃ étant choisi parmi un atome d'hydrogène et le groupe méthyle, et R₁ et R₂ représentent le groupe -CH₂CO₂H.

Parmi les ligands préférés, on trouve ceux ayant les formules suivantes : 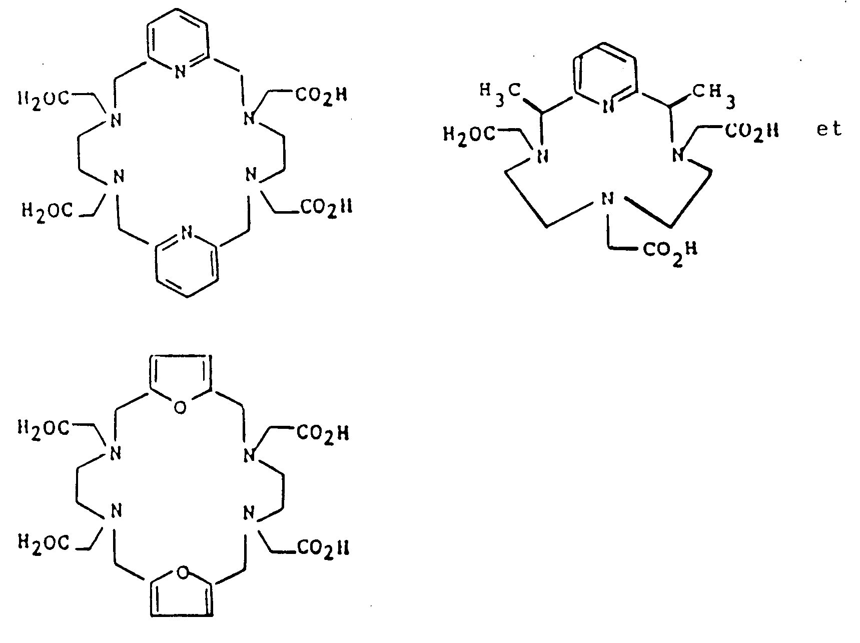 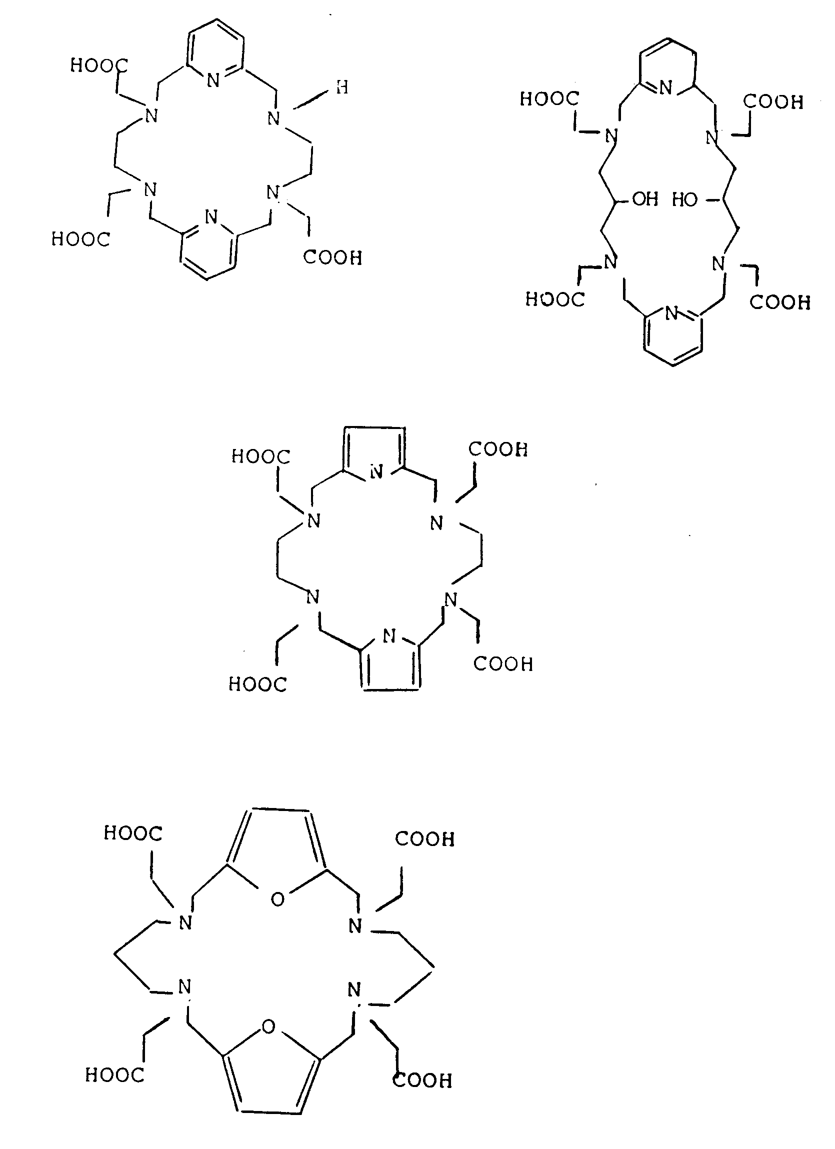
Les ligands peuvent être préparés par réaction d'une mole de polyamine de formule générale II 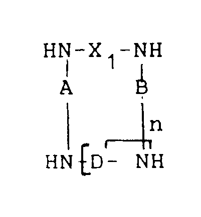
dans laquelle A, B et n sont tels que définis précédemment et D est choisi parmi les groupes A, B et un groupe X₁, X₁ étant choisi parmi les groupes 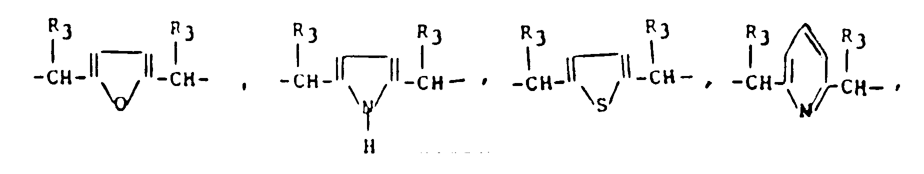
- soit 4 moles d'un composé de formule III

   Y-CH₂-CO₂H

   dans laquelle Y est un groupe labile tel qu'un atome de chlore, de brome ou d'iode ou un groupe mésyloxy ou tosyloxy, en présence de soude ou de potasse quand R₁ et R₂ signifient -CH₂CO₂H, ou 4 moles de formaldéhyde en présence d'acide phosphonique quand R₁ et R₂ signifient -CH₂PO₃H₂,
- soit 3 moles d'un composé de formule III tel que décrit ci-dessus en présence de soude ou de potasse quand R₁ représente -CH₂CO₂H, ou 3 moles de formaldéhyde ou l'acide phosphonique quand R₁ représente -CH₂PO₃H₂, puis éventuellement une mole d'un composé de formule IV

   Y-R₂

   dans laquelle Y est tel que défini précédemment et R₂ est choisi parmi un groupe alkyle en C₁-C₄, un groupe hydroxyalkyle en C₂-C₄,
Les polyamines cycliques de formule II peuvent être préparés par
a) cyclisation comme décrit par S.M. Nelson, Pure and Appl. Chem., 52, 2461-2476 (1980), par Fenton, Pure and Appl. Chem. 58, 1437-1444 (1986), par Khalil, K. Abid et al. dans Inorganica Chemica Acta, 82 (1984), 223-226, par K.F. Dancey et al. dans Synthetic communications, 16 (7), 795-801 (1986), par V. Mc Kee et al. dans J. Chem. Soc., Chem. Commun. 1983, 1465-1467 et par V. Mc Kee et al. dans J. Chem. Soc., Chem. Commun., 1985, 158-159, des composés dicarbonylés de formule V 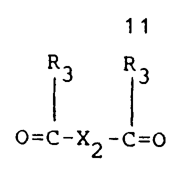 X₂ étant choisi parmi les groupes 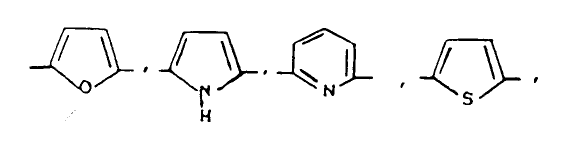 et R₃ étant tel que défini précedemment avec des polyamines de formule VI

   NH₂-A-NH₂

   et VII

   NH₂-B-NH₂

   ou avec une polyamine de formule VIII

   NH₂-A-NH-B-NH₂

   A et B étant tels que définis précédemment, et
b) réduction de la base de Schiff ainsi obtenue au moyen d'un réducteur métallique tel que Na BH₄ dans un solvant approprié tel que le méthanol pour obtenir les ligands de formule II.

La réaction de cyclisation se déroule en présence éventuellement d'un cation métallique approprié à la "cyclisation assistée par un métal", le métal étant choisi parmi les métaux de transition, les lanthanides, le baryum, le calcium et le strontium auquel cas, on élimine le cation métallique après la cyclisation au moyen d'un composé choisi parmi HBr KCN et H₂S.

Les composés de formule II peuvent également être préparés comme décrit dans "Darstellung und Komplexbildung von Polyazacycloakan essig-säure", H. Stetter et al., Tetrahedron, vol. 37, pp. 767 à 772, 1981 par cyclisation d'un composé de formule IX 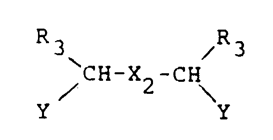
Y étant un groupe labile choisi parmi le chlore, le brome, l'iode, le groupe tosyloxy ou mésyloxy obtenus à partir des alcools correspondants avec un polysulfonamide de formule X : 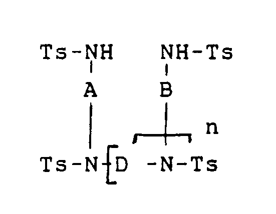
dans laquelle Ts est un groupe tosyle et A, D, B et n sont tels que définis précédemment en présence d'une base appropriée choisie parmi NaH, MeONa, EtONa et Cs₂CO₃ dans un solvant approprié tels que le diméthylformamide et le diméthylacétamide ou autres solvants aprotiques ou en présence d'un catalyseur de transfert de phase dans les conditions de transfert de phase connus de l'état de la technique.

La présente invention a en outre pour objet des complexes formés par les ligands de formule I avec des ions métalliques choisis parmi les ions des lanthanides, les ions des métaux de transition, le baryum, le bismuth et les radioisotopes 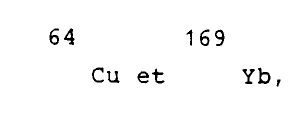
ainsi que les sels de ces complexes avec des bases minérales ou organiques pharmaceutiquement acceptables ou des aminoacides basiques.

Les complexes sont mono- ou polymétalliques de préférence mono- ou bi-métallique, neutres ou anioniques.

Dans ces complexes, les ions métalliques sont de préférence le gadolinium, l'europium, le dysprosium, le fer (Fe³⁺), le manganèse (Mn²⁺) et le baryum.

Un complexe bimétallique préféré est celui formé avec un ligand de formule I dans laquelle X et D représentent le groupe 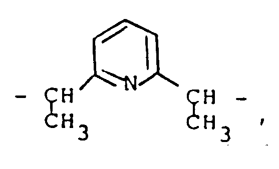
A et B représentent le groupe 2-hydroxy propylène, n = 1 et R₁ et R₂ représentent CH₂-COO⁻ avec deux ions Mn²⁺.

Comme exemples de sels on peut citer ceux formés avec l'hydroxyde de sodium, la N-méthylglucamine, la diéthanolamine, la lysine et l'arginine.

Les complexes peuvent être obtenus par réaction des ligands avec un sel ou un oxyde des métaux dans un solvant aqueux et éventuellement neutralisation pour former un sel.

Il va de soi que la présente invention englobe non seulement les ligands de formule I et les complexes précédemment définis sous forme de mélange racémique mais également les stéréoisomères de ces ligands et complexes.

Les complexes selon l'invention formés par les ligands de formule I peuvent être utilisés dans des applications diagnostiques in vitro et in vivo chez l'homme et chez l'animal.

En imagerie par résonance Magnétique et en Spectroscopie RMN in vivo, ils peuvent être utilisés comme agents de relaxation; à cet effet on préfère les complexes formés avec les métaux suivants : Gd³⁺, Mn²⁺ et Fe³⁺; comme agents de susceptibilité magnétique : à cet effet, on préfère les complexes formés avec les métaux Dy³⁺, Ho³⁺, Tb³⁺ et Er³⁺; ou comme agents de déplacement chimique : a cet effet on préfère les complexes formés avec les métaux Eu³⁺, Pr³⁺ et Yb³⁺.

Les complexes formés par les ligands de formule I et les métaux choisis de préférence parmi Gd³⁺, Er³⁺, Dy³⁺, Tb³⁺, Ce³⁺, La³⁺, Ba²⁺ peuvent être utilisés en imagerie par rayons X.

En médecine nucléaire, les complexes formés par les ligands de formule I et les métaux ⁶⁴Cu et ¹⁶⁹Yb, peuvent être utilisés dans des applications radiodiagnostiques et les complexes formés avec les métaux ⁶⁴Cu peuvent être utilisés dans des applications radiothérapeutiques après couplage du complexe à une biomolécule appropriée.

Les complexes formés par les ligands de formule I et les ions métalliques choisis de préférence parmi Eu³⁺ et Tb³⁺ peuvent être utilisés dans des applications diagnostiques in vitro par photoluminescence, telles que des fluoroimmuno-essais.

La présente invention a en conséquence également pour objet une composition de diagnostic administrable à l'homme caractérisé en ce qu'elle comprend au moins un complexe neutre ou anionique formé par un ligand de formule : 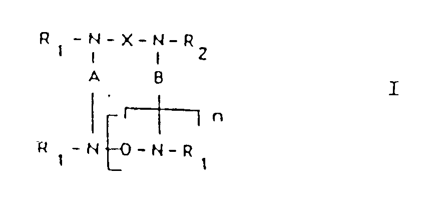
dans laquelle
A représente un groupe alkylène linéaire ou ramifié en C₂-C₅,
B représente un groupe alkylène linéaire ou ramifié en C₂-C₅,
n est égal à 0 ou 1,
D est choisi parmi un groupe alkylène linéaire ou ramifié en C₂-C₅ et un groupe X, X étant choisi parmi les groupes 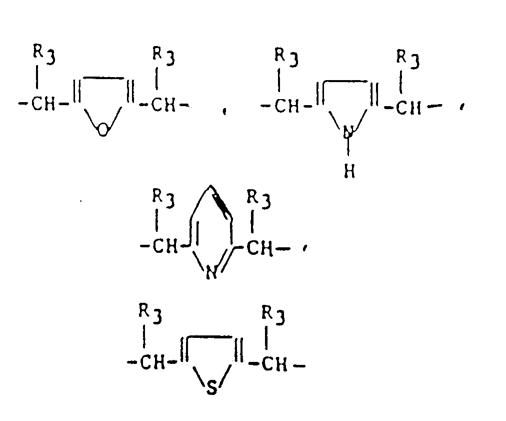
dans lesquels R₃ est choisi parmi un atome d'hydrogène et un groupe alkyle en C₁-C₃,
R₁ est choisi parmi les groupes -CH₂-COOH et -CH₂-PO₃H₂, et
R₂ est choisi parmi un atome d'hydrogène, un groupe alkyle en C₁-C₄, hydroxyalkyle en C₂-C₄ et un groupe R₁ tel que défini précédemment.

Outre les ligands précédemment décrits, la composition peut comprendre notamment un ligand de formule suivante: 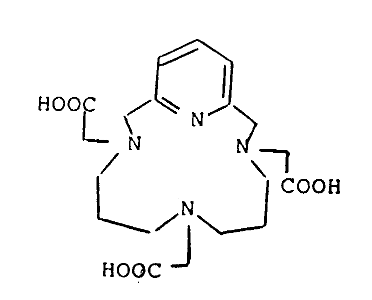
Ces compositions peuvent être constituées notamment par des solutions dans un solvant aqueux physiologiquement acceptable d'un complexe selon l'invention.

Les compositions de diagnostic selon l'invention peuvent être administrées :
- par voie parentérale dont la voie intra-veineuse, la voie intra-artérielle, la voie intra-lymphatique, la voie sous cutanée
- par voie orale
- par voie sous-arachnoïdienne
- par voie intrabronchique sous forme d'aérosol.

En imagerie par résonance magnétique, les doses sont très variables selon les voies d'administration.

Pour la voie intraveineuse ou intra-artérielle, la dose est d'environ 0,01 à 2 mM/kg.

Pour la voie orale cette dose peut aller jusqu'à 10 mM/kg.

Pour les autres voies d'administration, les doses utiles sont généralement inférieures à 1 mM/kg et même généralement pour la voie sous-arachnoïdienne inférieure à 0,05 mM/kg.

Dans l'utilisation comme agents de déplacement chimique pour la spectroscopie in vivo, comme agents de susceptibilité magnétique en IRM et comme agents de contraste en radiologie par rayons X les doses sont les mêmes, sauf par voie intraveineuse ou intra-artérielle où les doses peuvent être de 0,2 à 5 mM/kg.

Les exemples suivants illustrent la préparation des composés selon la présente demande.

Dans ces exemples :
- Les spectres RMN ont été réalisés sur un appareil Varian EM 360 à 60 MHz avec le TMs comme référence interne.

### Exemple 1 :

### Préparation du composé de formule :

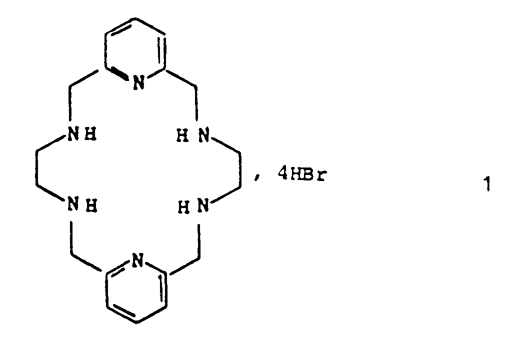

A une solution de diformyl pyridine (4,05g, 0,03 mole) et de BaCl₂,2H₂O (3,66g, 0,015 mole) dans 150 cm³ de méthanol, est ajoutée goutte à goutte, une solution de 1,2-diamino-éthane (0,03 mole) dans 20 cm³ de méthanol, le milieu réactionnel est chauffé à reflux pendant 3 heures.

Après refroidissement de la solution à 0°C, une première portion de NaBH₄ (0,08 mole) est ajoutée lentement, une seconde addition de NaBH₄ (0,04 mole) est effectuée 30 minutes après.

Le milieu réactionnel est agité pendant 1 heure 30 à température ambiante, concentré à sec et extrait avec CHCl₃.

L'huile obtenue après évaporation est recristallisée dans le méthanol sous forme de tétrabromhydrate.
. Rendement = 7,8 g (80%)
. RMN dans D₂O : 7,34 ppm (triplet, J = 8 Hz), 6,87 ppm (doublet, J = 8 Hz), 3,95 ppm (singulet), 3,24 ppm (singulet).

### Exemple 2 :

### Préparation du composé de formule :

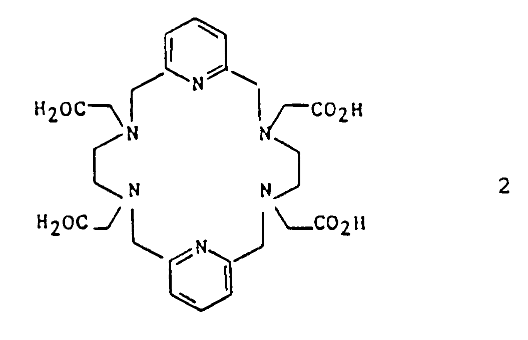

A une suspension de 1 (4,77 g, 0,007 mole) dans 10 cm³ d'eau, on ajoute une solution de soude (1,17 g dans 3 cm³ d'H₂O) en maintenant la température à 25°C.

On ajoute ensuite 15,28 g d'acide bromacétique, 9,53 g de 1 et une solution de soude (6,75 g dans 17 cm³ d'H₂O) alternativement par petites portions sur une période d'une heure. La température est maintenue en-dessous de 60°C. Après l'addition, le milieu réactionnel est ajusté à pH = 8,5 par addition de soude (4,5 g dans 10 cm³ d'H₂O) et maintenu à 45°C pendant 36 heures.

Le milieu réactionnel est ensuite ajusté à pH = 3 par addition de HBr puis chromatographié sur résine (Dowex 50 x 4 - 400).

Après lavage à l'eau de la résine, le produit est élué avec une solution d'ammoniaque 0,5 M.

Les fractions contenant le produit sont ajustées à pH2 par addition de HCl concentré et évaporées à sec. 15,4 g de produit sont récupérés avec un rendement de 94%.

¹H RMN dans D₂O : 7,54 ppm (triplet, J = 8 Hz), 7,04 ppm (doublet J = 8 Hz), 4,02 ppm (singulet), 3,15 ppm (singulet), 2,98 ppm (singulet)

### Exemple 3 :

### Préparation du sel de sodium du complexe Gd³ du composé de formule 2.

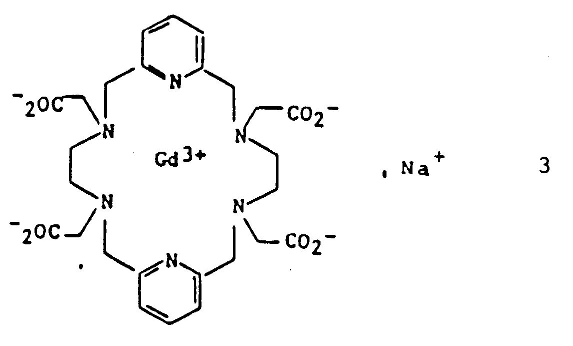

On chauffe à reflux pendant 4 heures une suspension de 0,3625 g de Gd₂O₃ et de 1,557 g du composé 2 dans 10 cm³ de H₂O. Après refroidissement à température ambiante, la solution limpide est neutralisée par addition de soude jusqu'à pH 7,2. Par addition d'acétone (150 cm³), on récupère 1,2g d'un solide blanc. Le complexe obtenu est recristallisé par dissolution dans un mélange eau/éthanol (40/60) et addition d'acétone.
. Analyse élémentaire - Calculé pour NaGdC₂₆H₃₁N₆O_{8,5} : C 41,95; H 4,17; N 11,29; Gd 21,14. Trouvé : C 42,15; H 4,30; N 11,25; Gd 20,47.

### Exemple 4 :

### Préparation du composé de formule

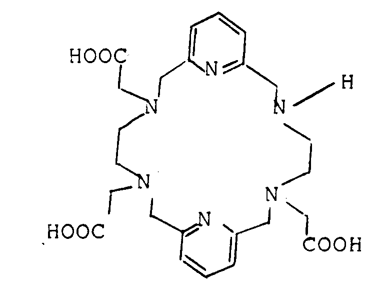

A une suspension du composé obtenu à l'exemple n° 1 (15 mmoles, 9,75 g) dans l'eau (30 ml) placée sous agitation est ajoutée lentement une solution de NaOH (60 mmoles, 2,6 g dans 10 ml H₂O) tandis que la température est maintenue aux alentours de 40° C au moyen d'un bain d'eau.

Le mélange est agité tandis que BrCH₂COOH solide (15 mmoles, 2,085 g) est ajouté. Le pH est maintenu aux alentours de 8,5 par addition continue d'une solution de NaOH (90 mmoles, 3,6 g dans 10 ml H₂O) et la température à 60° C pendant toute la durée de la réaction.

Après environ 3 heures, 15 mmoles (2,09 g) de BrCH₂COOH solide sont à nouveau ajoutés et après 4 heures supplémentaires, la quantité finale (15 mmoles, 2,09 g) de BrCH₂COOH solide est ajoutée. Pendant ces additions, le pH est maintenu à 8,5 et la température à 60° C.

Après 24 h, le pH du mélange réactionnel est ajusté à 3,0 par addition de HBr concentré.

Les sels et réactifs en excès sont éliminés du produit par chromatographie d'échange de cations (Dowex 50 x 8).

Le mélange réactionnel est chargé sur la colonne conditionnée et lavé avec de l'eau jusqu'à ce que l'éluat ne réagisse plus de manière positive aux halogénures et que le pH soit d'environ 4,5.

Le ligand est élué sur la colonne avec NH₃ aqueux 0,5 molaire. Les fractions ayant un pH de 4,1 à 5,1 sont rassemblées et évaporées à siccité sous pression réduite (rendement 6,0 g, 80 % de produit brut).

Le solide est dissous dans 60 ml de CH₃OH et ajouté goutte à goutte dans de l'acétone placée sous agitation (500 ml) pour obtenir un solide blanc très fin.

Le solide est récupéré et séché sous vide à température ambiante.

### Exemple 5 :

### Préparation du complexe de formule :

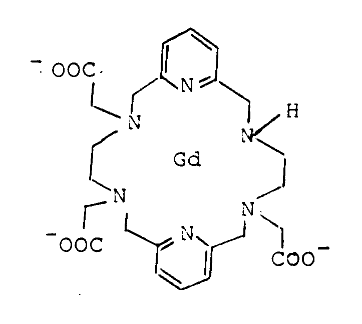

Gd₂O₃ (2,88 g, 8 mmoles) et le composé brut obtenu à l'exemple précédent (éluat évaporé obtenu à partir de la colonne échangeuse d'ions, 7,96 g, 16 mmoles) sont mis en suspension dans 100 ml d'H₂O à 80° C et agités pendant 24 heures. Durant ce temps la solution devient claire.

La solution est refroidie à température ambiante puis le solvant est chassé sous pression réduite.

Le solide résultant est dissous dans 15 ml d'H₂O et 5 ml de C₂H₅OH et ajouté goutte à goutte à 750 ml d'acétone placée sous agitation rapide.

Le précipité blanchâtre fin qui se forme est filtré puis lavé avec de l'acétone et séché sous vide à température ambiante.
Rendement : 6,8 g (66 %).

### Exemple 6 :

### Préparation du composé de formule :

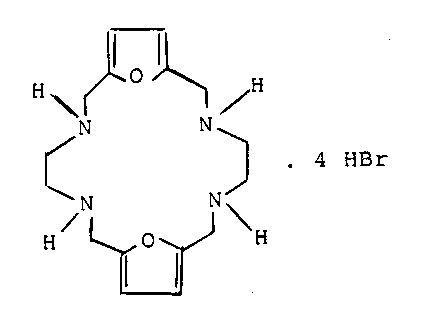

A une solution de 2,5-furanedicarbaldéhyde (0,505 g, 4 mmoles) et de Ba(SCN)₂.3H₂O (0,615 g, 2 mmoles) dans 60 ml MeOH, placée sous agitation, est ajoutée goutte à goutte une solution de 1,2-diamino éthane (0,270 ml, 4 mmoles dans 40 ml MeOH) sur une période de 15 minutes.

Le mélange est agité pendant 3 heures et la couleur vire au jaune orangé.

Le mélange est alors refroidi dans un bain de glace tandis que NaBH₄ (0,404 g, 10,7 mmoles) est ajouté lentement.

La couleur de la réaction vire au jaune citron.

Après une agitation de 30 minutes, une autre addition de NaBH₄ (0,2018 g, 5,35 mmoles) est effectuée.

Après une agitation de 2 heures à température ambiante, le mélange est évaporé à siccité sous pression réduite et le résidu (un mélange de solides jaunes et blancs) est extrait à cinq reprises, avec à chaque fois 25 ml de CHCl₃.

Les extraits chloroformiques réunis sont filtrés et évaporés sous pression réduite pour donner une huile jaune.

L'huile est dissoute dans MeOH (10 à 15 ml) et HBr (6 à 7 gouttes d'une solution aqueuse à 48 %) est ajouté goutte à goutte à cette solution. Le produit précipite sous forme d'un solide jaune pâle.

Le produit est récupéré puis séché sous vide à température ambiante.
Rendement : 0,6 g ou 42 %.
Analyse élémentaire pour C₁₆H₃₁N₄O_{3,5}Br₄ : calculé : C, 29,33 ;
H, 4,77 ; N, 8,55 ; Br, 48,79. Trouvée: C, 29,24 ; H, 4,81 ; N, 8,56 ; Br, 48,00.

### Exemple 7 :

### Préparation du composé de formule :

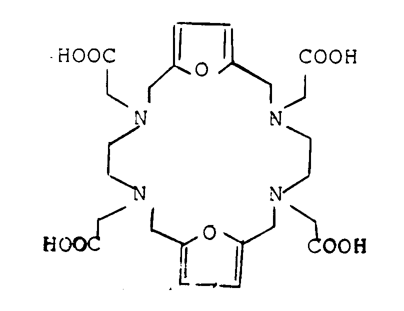

A une suspension du composé obtenu à l'exemple précédent (0,01 mole, 6,28 g) dans de l'eau (30 ml) placée sous agitation, est ajoutée lentement une solution de NaOH (1,6 g, 0,04 mole dans 20 ml d'H₂O) tandis que la température est maintenue aux alentours de 30° au moyen d'un bain d'eau.

Le mélange est agité tandis que BrCH₂COOH solide (0,02 mole, 2,78 g) est ajouté. Le pH est maintenu aux alentours de 8,5 par addition continue d'une solution de NaOH (3,2 g dans 10 ml H₂O) et la température à 60° C pendant toute la durée de la réaction.

Après environ 3,5 heures, 0,010 mole (1,39 g de BrCH₂COOH solide est à nouveau ajouté et après 3,5 heures supplémentaires, la quantité finale (0,010 mole, 1,39 g) de BrCH₂COOH solide est ajoutée. Durant ces additions, le pH était à 8,5 et la température maintenue à 60° C.

Après 24 heures, la consommation de la solution de NaOH a cessé et la réaction est arrêtée. Le pH du mélange réactionnel est ajusté à 2,7 par addition de HBr concentré.

Les sels et réactifs en excès sont éliminés du produit par chromatographie d'échange de cations (Dowex 50 x 8).

Le mélange réactionnel est chargé sur la colonne conditionnée et lavé avec de l'eau jusqu'à ce que l'éluat ne réagisse plus positivement aux halogénures et que le pH soit d'environ 4,5.

Le ligand est élué de la colonne avec NH₃ aqueux 0,5 M. Les fractions ayant un PH égal à 3,2 sont rassemblées et évaporées à siccité sous pression réduite (rendement 2,7 g).

Le solide est dissous dans 15 ml de CH₃OH et ajouté goutte à goutte dans 200 ml d'acétone placée sous agitation pour obtenir un solide blanc très fin.

Le solide est récolté et séché sous vide à température ambiante.
Rendement 1,8 g, 50 %.
Analyse élémentaire pour C₂₄H₃₃N₄O_{10.5} ; Calc. C, 52,84; H, 6,10; N, 10,27. Trouvé C, 53,06; H, 6,18; N, 9,67.

### Exemple 8 :

### Préparation du complexe de formule :

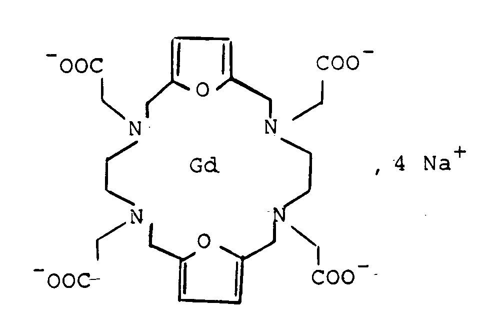

Gd₂(CO₃)₂ (0,3g, 0,6 mmole) et le composé obtenu à l'exemple précédent (0,64 g, 1,2 mmole) sont mis en suspension dans 40 ml d'H₂O à température ambiante et agités pendant 48 heures.

La suspension est filtrée pour donner une solution incolore qui est neutralisée à pH 7 avec une solution de NaOH (0,05 g dans 5 ml d'H₂O). Le solvant est chassé sous pression réduite.

Le solide résultant est dissous dans 5 ml d'H₂O et 5 ml de C₂H₅OH et est ajouté goutte à goutte à 200 ml d'acétone placée sous agitation rapide.

Le précipité blanc fin qui se forme est récupéré par filtration à la trompe à eau, lavé avec de l'acétone et séché sous vide à température ambiante.
Rendement 0,56 g (65 %).

### Exemple 9 :

### Préparation du composé de formule :

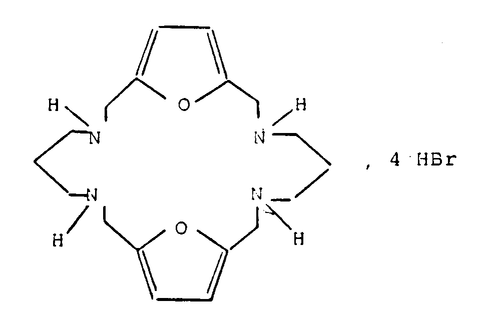

A une solution de 2,5-furanedicarbaldéhyde (0,505 g, 4 mmoles) et de Ba(SCN)₂.3H₂O (0,615 g, 2 mmoles) dans 60 ml de MeOH, placée sous agitation, est ajoutée goutte à goutte une solution de 1,3-diamino-propane (0,340 ml, 4 mmoles dans 40 ml MeOH) pendant une durée de 15 mn.

Le mélange est agité pendant 20 mn et la couleur vire au jaune orangé.

Le mélange est alors refroidi dans un bain de glace pendant que NaBH₄ (0,404 g, 10,7 mmoles) est ajouté lentement.

La couleur de la réaction vire au jaune citron.

Après une agitation pendant 30 mn, une autre addition de NaBH₄ (0,2018 g, 5,35 mmoles) est effectuée.

Après une agitation de 2 heures à température ambiante, le mélange est évaporé à siccité sous pression réduite et le résidu (un mélange de solides jaunes et blancs) est extrait à quatre reprises avec à chaque fois 15 ml de CHCl₃.

Les extraits de chloroforme réunis sont filtrés et évaporés sous pression réduite pour fournir une huile orange.

L'huile est dissoute dans du méthanol (10 à 15 ml) et HBr (6 à 7 gouttes d'une solution aqueuse à 48 %) est ajouté goutte à goutte à cette solution. Le produit précipite sous forme d'un solide jaune pâle.

Le produit est filtré et séché sous vide à température ambiante.
Rendement : 0,67 g, 50 %.
Analyse élémentaire : calculé pour C₁₈H₃₂N₄O₂Br₄ : C, 32,95 ; H, 4,92; N, 8,54; Br, 48,74. Trouvé : C, 33,04; H, 4,96; N, 8,36; Br, 48,28.

### Exemple 10 :

### Préparation du composé de formule :

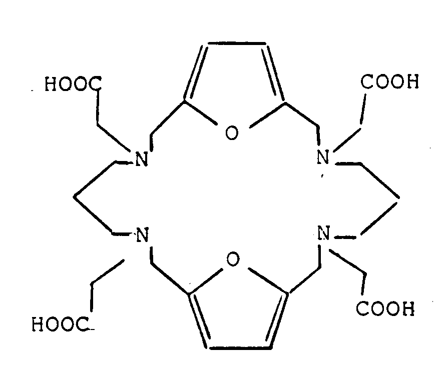

On prépare ce composé selon le mode opératoire décrit dans les exemples 2 et 7.

### Exemple 11 :

### Préparation du composé de formule :

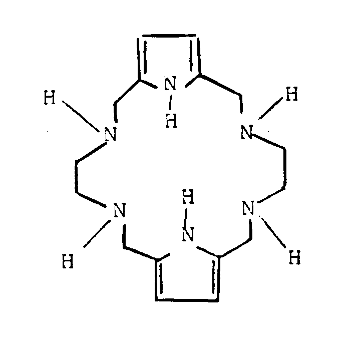

A une solution de 2,5-pyrroledicarbaldéhyde (1,00 g, 8 mmoles) et de BaCl₂.2H₂O (0,976g, 4 mmoles) dans 150 ml de méthanol placée sous agitation, est ajoutée goutte à goutte une solution de 1,2-diaminoéthane (0,48 g, 8 mmoles dans 20 ml MeOH) pendant une durée de 15 mn.

La solution initialement jaune vire à l'orange au cours de l'addition d'éthylènediamine. Le mélange est chauffé à reflux pendant 3 heures et la couleur vire au brun foncé.

Le mélange est alors refroidi dans un bain de glace pendant que NaBH₄ (0,805 g, 21,3 mmoles) est ajouté lentement. La réaction devient brun clair en formant un précipité blanc.

Après une agitation de 30 mn, une autre addition de NaBH₄ (0,404 g, 10,7 mmoles) est effectuée.

Après une agitation de 1,5 heure à température ambiante, le mélange est évaporé à siccité sous pression réduite et le résidu (un mélange d'huile orange et de solide blanc) est extrait à quatre reprises avec à chaque fois 75 ml de CHCl₃.

Les extraits chloroformiques réunis sont filtrés et évaporés sous pression réduite pour fournir une huile orange.

L'huile est séchée sous vide à température ambiante pour fournir un solide brun clair.
Rendement 49 %.
¹H RMN dans CDCl₃ : 10,08 ppm (large singulet, 2H, pyrrole NH), 5,95 ppm (singulet, 4H, pyrrole CH), 3,80 ppm (singulet, 8H, CH₂), 2,70 ppm (singulet, 8H, CH₂).

### Exemple 12

### Préparation du composé de formule

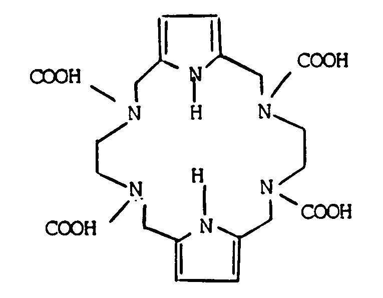

On prépare ce composé selon le mode opératoire décrit dans les exemples 2 et 7.

### Exemple 13 :

### Préparation du complexe de formule :

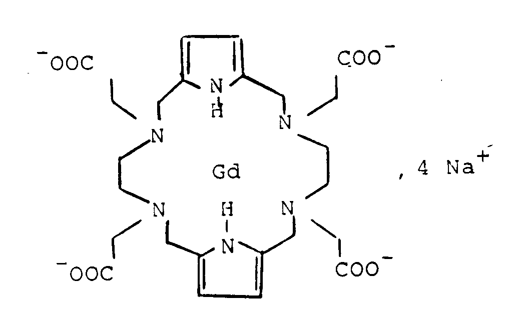

On prépare ce complexe selon le mode opératoire décrit dans les exemples 3 et 5 précédents.

### Exemple 14 :

### Préparation du composé de formule :

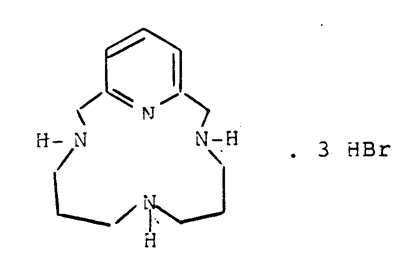

A une solution de 2,6-pyridinedicarbaldéhyde (1,35 g, 0,01 mole) et de triflate de zinc (3,63 g, 0,01 mole) dans 25 ml de C₂H₅OH et 25 ml d'H₂O placée sous agitation, est ajoutée goutte à goutte une solution d'iminobis (propylamine) (1,4 ml, 0,01 mole dans 10 ml C₂H₅OH) pendant une durée de 15 mn.

Du NaBr (5,5 g) est ajouté au mélange réactionnel. Le mélange est agité pendant 4 heures pendant lesquelles le produit se dépose. Le produit est récupéré par filtration à la trompe à eau et est séché sous vide à température ambiante pendant 12 heures. Le produit obtenu (1,03 g, 1,7 mmole) est dissous dans 50 ml de méthanol pendant que NaBH₄ (0,48 g, 13 mmoles) est ajouté lentement.

Après une agitation pendant 40 mn, une autre addition de NaBH₄ (0,44 g, 12 mmoles) est effectuée.

Après avoir agité pendant 2 heures à température ambiante, le mélange est évaporé à siccité sous pression réduite et le résidu (un mélange de solides blanchâtre) est extrait à quatre reprises avec à chaque fois 25 ml de CHCl₃. Les extraits chloroformiques réunis sont filtrés et évaporés sous pression reduite pour fournir un solide de couleur crème.

Le solide est dissous dans du méthanol (60 ml) et HBr (environ 3 ml d'une solution aqueuse à 48 %) est ajouté goutte à goutte à la solution placée sous agitation pour précipiter le produit à partir du milieu acide sous la forme d'un solide jaune pâle.

Le produit est récolté par filtration à la trompe à eau et est séché sous vide à température ambiante.
Rendement : 0,52 g, 63 %.
¹H RMN dans D₂O : 7,93 ppm (triplet, aire 1), 7,50 ppm (doublet, aire 2), 4,47 ppm (singulet, aire 4), 3,05-3,35 ppm (multiplet, aire 8), 2,25 ppm (multiplet, aire 4).

### Exemple 15 :

### Préparation du composé de formule :

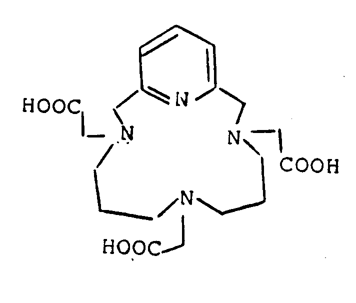

On prépare ce composé par réaction avec l'acide bromoacétique en présence de NaOH selon le mode opératoire décrit à l'exemple 5 précédent.

De la même manière, on prépare les complexes de gadolinium des ligands correspondant aux exemples 10 et 15 en présence d'une quantité stoechiométrique de Gd₂O₃ en milieu aqueux selon le mode opératoire décrit dans l'exemple 8.

## Revendications (Revendications pour l'(les) Etat(s) contractant(s) suivant(s): AT, BE, CH, DE, DK, FR, GB, GR, IT, LI, LU, NL, SE)

1. Ligands de formule : 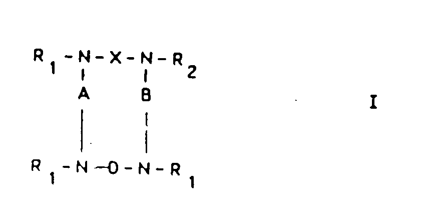 dans laquelle
A représente un groupe alkylène linéaire ou ramifié en C₂-C₅,
B représente un groupe alkylène linéaire ou ramifié en C₂-C₅,
D représente un groupe X, X étant choisi parmi les groupes 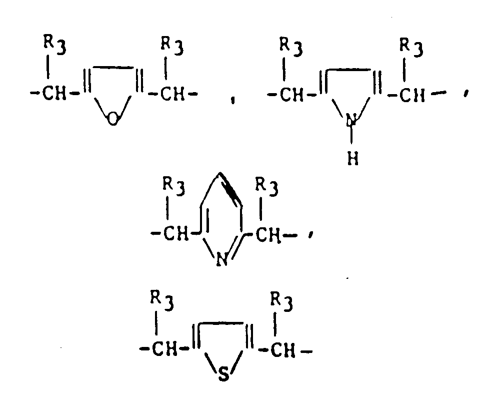 dans lesquels R₃ est choisi parmi un atome d'hydrogène et un groupe alkyle en C₁-C₃,
R₁ est choisi parmi les groupes -CH₂-COOH et
-CH₂-PO₃H₂, et
R₂ est choisi parmi un atome d'hydrogène, un groupe alkyle en C₁-C₄, hydroxyalkyle en C₂-C₄ et un groupe R₁ tel que défini précédemment.

2. Ligands selon la revendication 1, caractérisés en ce que R₂ est choisi parmi un atome d'hydrogène, un groupe hydroxyalkyle en C₂-C₄ et un groupe R₁ tel que défini à la revendication 1.

3. Ligands selon l'une quelconque des revendications 1 ou 2, dans lesquelles A et B sont identiques et choisis parmi les groupes éthylène et n-propylène, X et D sont identiques et choisis parmi les groupes 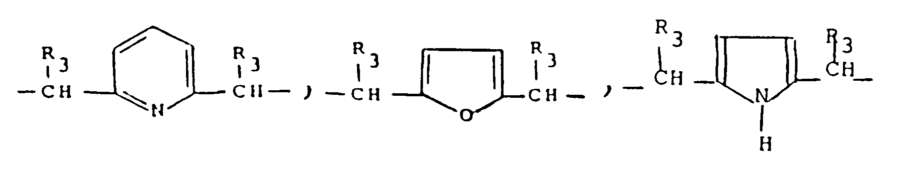 R₃ étant choisi parmi un atome d'hydrogène et le groupe méthyle, et R₁ et R₂ représentent le groupe -CH₂-CO₂H.

4. Ligand selon la revendication 1 ou 2, dans laquelle A et B représentent un groupe -CH₂-CH₂-, X et D représentent le groupe 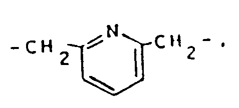 et R₁ et R₂ représentent le groupe -CH₂-COOH.

5. Ligand selon la revendication 1 ou 2, dans laquelle A et B représentent le groupe -CH₂-CH₂-, X et D représentent le groupe 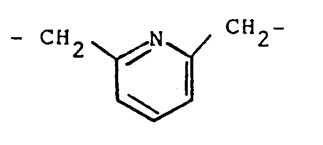 R₁ représente le groupe -CH₂-COOH et R₂ représente l'atome d'hydrogène.

6. Ligand selon la revendication 1 ou 2, dans laquelle A et B représentent le groupe -CH₂-CH₂-, X et D représentent le groupe 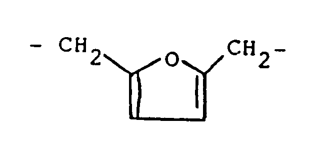 et R₁ et R₂ représentent le groupe -CH₂-COOH.

7. Ligand selon la revendication 1 ou 2, dans laquelle A et B représentent le groupe -(CH₂)₃-, X et D représentent le groupe 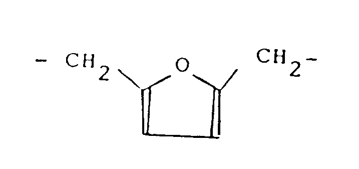 et R₁ et R₂ représentent le groupe -CH₂-COOH.

8. Ligand selon la revendication 1 ou 2, dans laquelle A et B représentent le groupe -CH₂-CH₂, X et D représentent le groupe 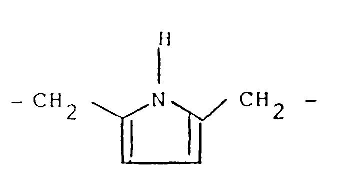 et R₁ et R₂ représentent le groupe -CH₂-COOH.

9. Procédé de préparation d'un ligand selon l'une quelconque des revendications 1 ou 2, caractérisé en ce que l'on fait réagir une polyamine cyclique de formule II 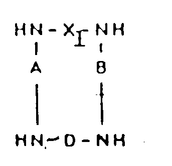 dans laquelle A, B ont la signification donnée à la revendication 1, D représente le groupe X₁, X₁ étant choisi parmi les groupes 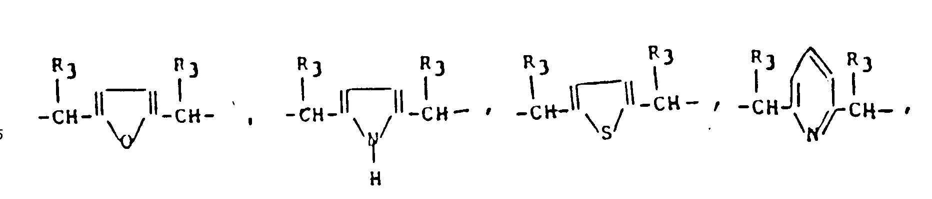 R₃ étant tel que défini à la revendication 1,
avec un composé de formule III
Y-CH₂-COOH
dans laquelle Y est un groupe labile en présence de NaOH ou de KOH quand R₁ a la signification -CH₂-COOH ou avec la formaldéhyde en présence d'acide phosphonique quand R₁ a la signification -CH₂-PO₃H₂, et éventuellement un composé de formule IV
Y-R₂
dans laquelle Y est un groupe labile et R₂ est tel que défini à la revendication 1 ou la revendication 2.

10. Complexes neutres ou anioniques formés par des ligands de formule I selon la revendication 1, avec des ions métalliques choisis parmi les ions des lanthanides, les ions des métaux de transition, l'ion baryum, l'ion bismuth et les radioisotopes suivants :
⁶⁴Cu et ¹⁶⁹Yb,
ainsi que les sels de ces complexes avec des bases minérales ou organiques pharmaceutiquement acceptables ou des aminoacides basiques.

11. Complexes selon la revendication 10, dans lesquels l'ion métallique est choisi parmi le gadolinium, l'europium, le dysprosium, le fer (Fe³⁺), le manganèse (Mn²⁺) et le baryum.

12. Composition de diagnostic administrable à l'homme comprenant un complexe neutre ou anionique formé par un ligand de formule : 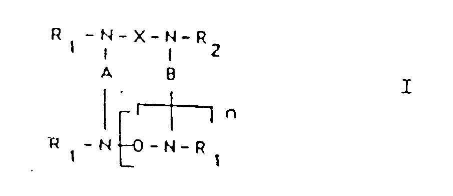 dans laquelle
A représente un groupe alkylène linéaire ou ramifié en C₂-C₅,
B représente un groupe alkylène linéaire ou ramifié en C₂-C₅,
n est égal à 0 ou 1,
D est choisi parmi un groupe alkylène linéaire ou ramifié en C₂-C₅ et un groupe X, X étant choisi parmi les groupes 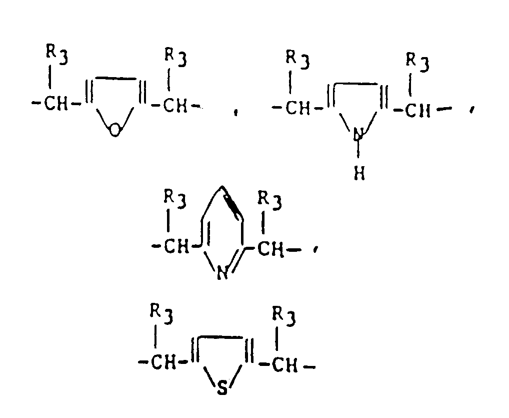 dans lesquels R₃ est choisi parmi un atome d'hydrogène et un groupe alkyle en C₁-C₃,
R₁ est choisi parmi les groupes -CH₂-COOH et -CH₂-PO₃H₂, et
R₂ est choisi parmi un atome d'hydrogène, un groupe alkyle en C₁-C₄, hydroxyalkyle en C₂-C₄ et un groupe R₁ tel que défini précédemment.

13. Composition selon la revendication 12, caractérisée en ce que R₂ est choisi parmi un atome d'hydrogène, un groupe hydroxyalkyle en C₂-C₄ et un groupe R₁ tel que défini à la revendication 1.

14. Composition selon la revendication 12, caractérisée en ce que dans le ligand de formule I n est égal à 1 et D représente le groupe X tel que défini dans la revendication 1.

15. Composition selon l'une quelconque des revendications 12 ou 13, caractérisée en ce que dans le ligand de formule I, A et B sont identiques et choisis parmi les groupes éthylène et n-propylène, n est égal à 1, X et D sont identiques et choisis parmi les groupes 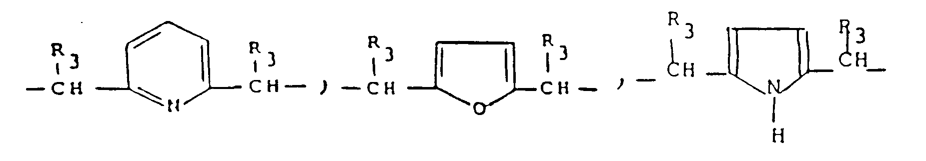 R₃ étant choisi parmi un atome d'hydrogène et le groupe méthyle, et R₁ et R₂ représentent le groupe -CH₂-CO₂H.

16. Composition selon la revendication 12 ou 13, caractérisée en ce que dans le ligand de formule I A et B sont identiques et choisis parmi les groupes éthylène et n-propylène, n est égal à 0, X est choisi parmi les groupes 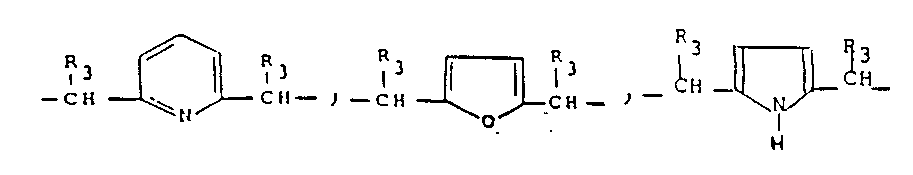 R₃ étant choisi parmi un atome d'hydrogène et le groupe méthyle, R₁ représente le groupe -CH₂-CO₂H et R₂ représente un atome d'hydrogène ou le groupe -CH₂-CO₂H.

17. Composition selon la revendication 12 ou 13, caractérisée en ce que dans le ligand de formule I A et B représentent un groupe -CH₂-CH₂-, n = 1, X et D représentent le groupe 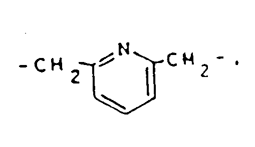 et R₁ et R₂ représentent le groupe -CH₂-COOH.

18. Composition selon la revendication 12 ou 13, caractérisée en ce que dans le ligand de formule I A et B représentent le groupe -CH₂-CH₂-, n = 1, X et D représentent le groupe 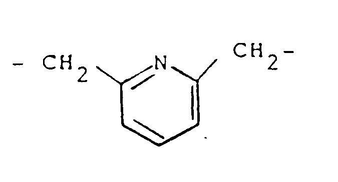 R₁ représente le groupe -CH₂-COOH et R₂ représente l'atome d'hydrogène.

19. Composition selon la revendication 12 ou 13, caractérisée en ce que dans le ligand de formule I A et B représentent le groupe -CH₂-CH₂-, n = 1, X et D représentent le groupe 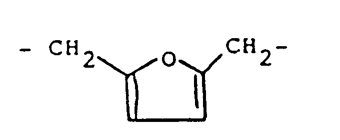 et R₁ et R₂ représentent le groupe -CH₂-COOH.

20. Composition selon la revendication 12 ou 13, caractérisée en ce que dans le ligand de formule I A et B représentent le groupe -(CH₂)₃-, n = 1, X et D représentent le groupe 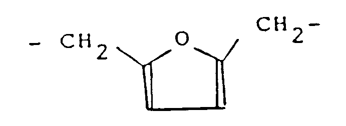 et R₁ et R₂ représentent le groupe -CH₂-COOH.

21. Composition selon la revendication 12 ou 13, caractérisée en ce que dans le ligand de formule I A et B représentent le groupe -CH₂-CH₂, n = 1, X et D représentent le groupe 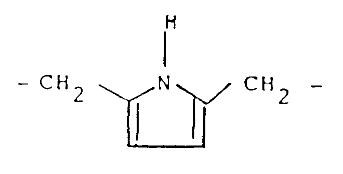 et R₁ et R₂ représentent le groupe -CH₂-COOH.

22. Composition selon la revendication 12, comprenant un complexe métallique dans lequel l'ion métallique est choisi parmi le gadolinium, l'europium, le dysprosium, le fer (Fe³⁺), le manganèse (Mn²⁺), et le baryum.

23. Composition selon l'une quelconque des revendications précédentes, constituée par une solution dans un solvant aqueux.

24. Composition de diagnostic selon l'une quelconque des revendications 12 à 22, dans laquelle le métal est choisi parmi Gd³⁺, Mn²⁺, Fe³⁺, Dy³⁺, Pr³⁺, Eu³⁺, Tb³⁺ et Yb³⁺, pour l'imagerie par résonance magnétique.

25. Composition selon l'une quelconque des revendications 12 à 22 dans laquelle le métal est choisi parmi Gd³⁺, Er³⁺, Dy³⁺, Ce³⁺, La³⁺, Bi²⁺ et Ba²⁺ pour l'imagerie par rayons X.

26. Utilisation d'un complexe selon l'une quelconque des revendications 10 ou 11, pour la préparation d'une composition de diagnostic administrable à l'homme destinée à l'imagerie par résonance magnétique.

27. Utilisation d'un complexe selon l'une quelconque des revendications 10 ou 11, pour la préparation d'une composition de diagnostic administrable à l'homme destinée à l'imagerie par rayons X.

## Revendications (Revendications pour l'(les) Etat(s) contractant(s) suivant(s): ES)

1. Procédé de préparation d'un ligand de formule : 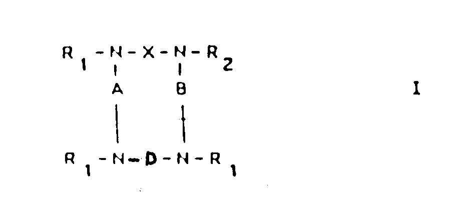 dans laquelle
A représente un groupe alkylène linéaire ou ramifié en C₂-C₅,
B représente un groupe alkylène linéaire ou ramifié en C₂-C₅,
D représente un groupe X, X étant choisi parmi les groupes 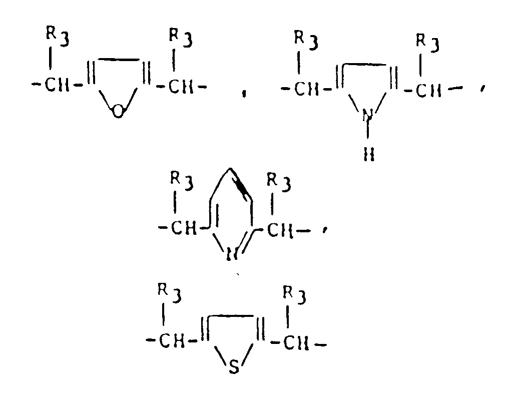 dans lesquels R₃ est choisi parmi un atome d'hydrogène et un groupe alkyle en C₁-C₃,
R₁ est choisi parmi les groupes -CH₂-COOH et
-CH₂-PO₃H₂, et
R₂ est choisi parmi un atome d'hydrogène, un groupe alkyle en C₁-C₄, hydroxyalkyle en C₂-C₄ et un groupe R₁ tel que défini précédemment,
caractérisé en ce qu'on fait réagir une polyamine cyclique de formule II 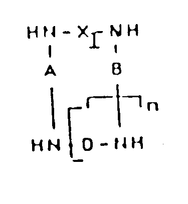 dans laquelle A, B ont la signification donnée à la revendication 1, D représente le groupe X₁, X₁ étant choisi parmi les groupes 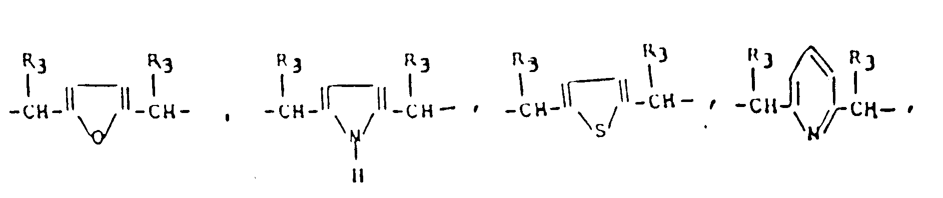 R₃ étant tel que défini à la revendication 1, n est égal à 1, avec un composé de formule III
Y-CH₂-COOH
dans laquelle Y est un groupe labile en présence de NaOH ou de KOH quand R₁ a la signification -CH₂-COOH ou avec la formaldéhyde en présence d'acide phosphonique quand R₁ a la signification -CH₂-PO₃H₂, et éventuellement un composé de formule IV
Y-R₂
dans laquelle Y est un groupe labile et R₂ est tel que défini précédemment.

2. Procédé selon la revendication 1, caractérisé en ce que l'on prépare un ligand dans lequel R₂ est choisi parmi un atome d'hydrogène, un groupe hydroxyalkyle en C₂-C₄ et un groupe R₁ tel que défini à la revendication 1.

3. Procédé selon la revendication 1 ou 2, caractérisé en ce que l'on prépare un ligand de formule I dans laquelle A et B sont identiques et choisis parmi les groupes éthylène et n-propylène, X et D sont identiques et choisis parmi les groupes 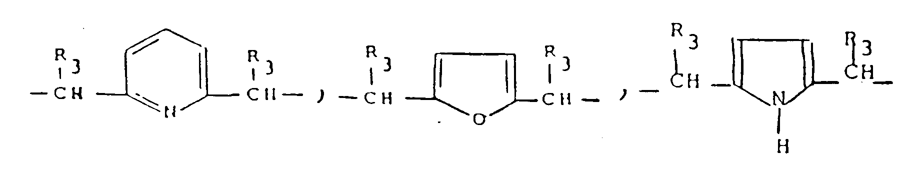 R₃ étant choisi parmi un atome d'hydrogène et le groupe méthyle, et R₁ et R₂ représentent le groupe -CH₂-CO₂H.

4. Procédé selon la revendication 1 ou 2, caractérisé en ce que l'on prépare un ligand de formule I dans laquelle A et B représentent un groupe -CH₂-CH₂-, X et D représentent le groupe 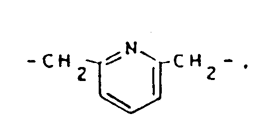 et R₁ et R₂ représentent le groupe -CH₂-COOH.

5. Procédé selon la revendication 1 ou 2, caractérisé en ce que l'on prépare un ligand de formule I dans laquelle A et B représentent le groupe -CH₂-CH₂-, X et D représentent le groupe 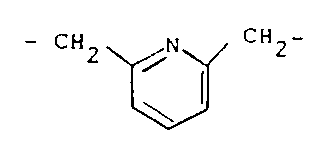 R₁ représente le groupe -CH₂-COOH et R₂ représente l'atome d'hydrogène.

6. Procédé selon la revendication 1 ou 2, caractérisé en ce que l'on prépare un ligand de formule I dans laquelle A et B représentent le groupe -CH₂-CH₂-, X et D représentent le groupe 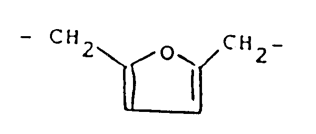 R₁ et R₂ représentent le groupe -CH₂-COOH.

7. Procédé selon la revendication 1 ou 2, caractérisé en ce que l'on prépare un ligand de formule I dans laquelle A et B représentent le groupe -(CH₂)₃-, X et D représentent le groupe 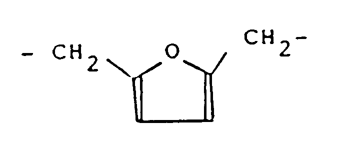 R₁ et R₂ représentent le groupe -CH₂-COOH.

8. Procédé selon la revendication 1 ou 2, caractérisé en ce que l'on prépare un ligand de formule I dans laquelle A et B représentent le groupe -CH₂-CH₂-, X et D représentent le groupe 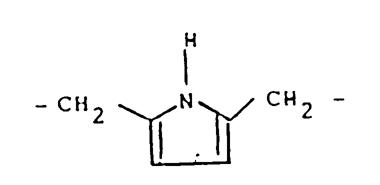 R₁ et R₂ représentent le groupe -CH₂-COOH.

9. Procédé de préparation de complexes mono-ou polymétalliques neutres ou anioniques, caractérisé en ce que l'on fait réagir un ligand préparé selon l'une quelconque des revendications 1 à 8 avec des ions métalliques choisis parmi les ions des lanthanides, les ions des métaux de transition, l'ion baryum, l'ion bismuth et les radioisotopes suivants :
⁶⁴Cu et ¹⁶⁹Yb,
puis éventuellement avec des bases minérales ou organiques pharmaceutiquement acceptables ou des aminoacides basiques pour obtenir les sels de ces complexes.

10. Procédé selon la revendication 9, caractérisé en ce qu'on prépare des compleses métalliques, dans lesquels l'ion métallique est choisi parmi le gadolinium, l'europium, le dysprosium, le fer (Fe³⁺), le manganèse (Mn²⁺) et le baryum.

11. Procédé de préparation d'une composition de diagnostic administrable à l'homme caractérisé en ce que l'on mélange un complexe métallique, neutre ou anionique formé par un ligand de formule 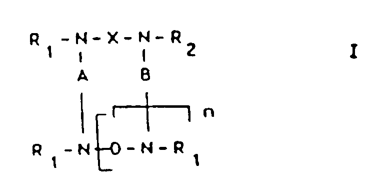 dans laquelle
A représente un groupe alkylène linéaire ou ramifié en C₂-C₅,
B représente un groupe alkylène linéaire ou ramifié en C₂-C₅,
n est égal à 0 ou 1,
D est choisi parmi un groupe alkylène linéaire ou ramifié en C₂-C₅ et un groupe X, X étant choisi parmi les groupes 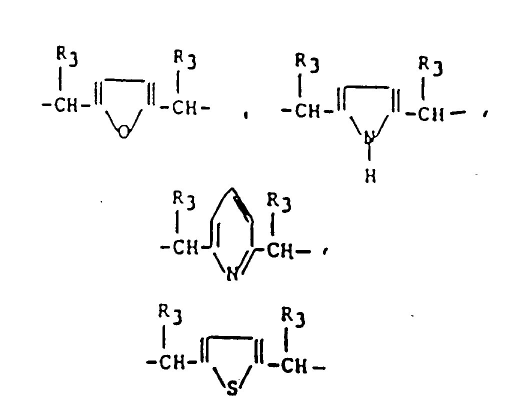 dans lesquels R₃ est choisi parmi un atome d'hydrogène et un groupe alkyle en C₁-C₃,
R₁ est choisi parmi les groupes -CH₂-COOH et -CH₂-PO₃H₂, et
R₂ est choisi parmi un atome d'hydrogène, un groupe alkyle en C₁-C₄, hydroxyalkyle en C₂-C₄ et un groupe R₁ tel que défini précédemment ou un sel d'un complexe tel que défini ci-dessus avec une base minérale ou organique pharmaceutiquement acceptable ou un aminoacide basique, avec un excipient pharmaceutiquement acceptable.

12. Procédé selon la revendication 11, caractérisé en ce que R₂ est choisi parmi un atome d'hydrogène, un groupe hydroxyalkyle en C₂-C₄ et un groupe R₁ tel que défini à la revendication 1.

13. Procédé selon la revendication 11, caractérisé en ce que dans le ligand de formule I n est égal à 1 et D représente le groupe X tel que défini dans la revendication 1.

14. Procédé selon l'une quelconque des revendications 11 ou 12, caractérisé en ce que dans le ligand de formule I, A et B sont identiques et choisis parmi les groupes éthyléne et n-propylène, n est égal à 1, X et D sont identiques et choisis parmi les groupes 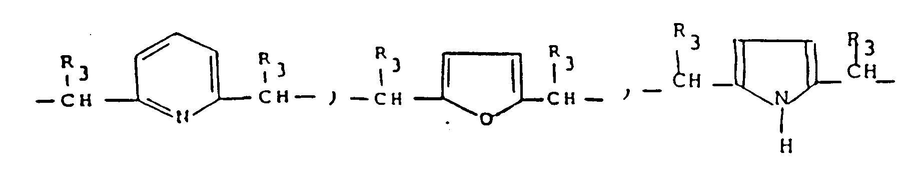 R₃ étant choisi parmi un atome d'hydrogène et le groupe méthyle, et R₁ et R₂ représentent le groupe -CH₂-CO₂H.

15. Procédé selon la revendication 11 ou 12, caractérisé en ce que dans le ligand de formule I A et B sont identiques et choisis parmi les groupes éthylène et n-propylène, n est égal à 0, X est choisi parmi les groupes 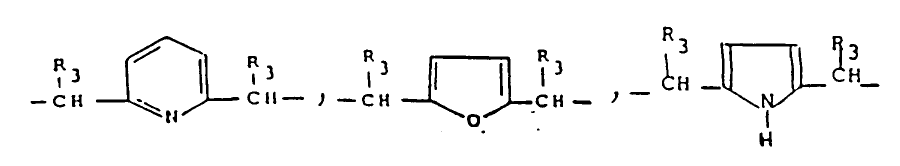 R₃ étant choisi parmi un atome d'hydrogène et le groupe méthyle, R₁ représente le groupe -CH₂-CO₂H et R₂ représente un atome d'hydrogène ou le groupe -CH₂-CO₂H.

16. Procédé selon la revendication 11 ou 12, caractérisé en ce que dans le ligand de formule I A et B représentent un groupe -CH₂-CH₂- n = 1, X et D représentent le groupe 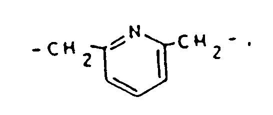 et R₁ et R₂ représentent le groupe -CH₂-COOH.

17. Procédé selon la revendication 11 ou 12, caractérisé en ce que dans le ligand de formule I A et B représentent le groupe -CH₂-CH₂-, n = 1, X et D représentent le groupe 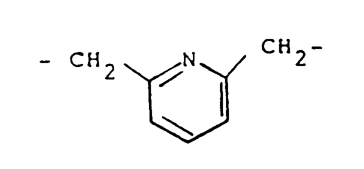 R₁ représente le groupe -CH₂-COOH et R₂ représente l'atome d'hydrogène.

18. Procédé selon la revendication 11 ou 12, caractérisé en ce que dans le ligand de formule I A et B représentent le groupe -CH₂-CH₂-, n = 1, X et D représentent le groupe 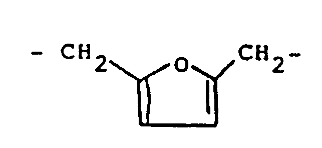 et R₁ et R₂ représentent le groupe -CH₂-COOH.

19. Procédé selon la revendication 11 ou 12, caractérisé en ce que dans le ligand de formule I A et B représentent le groupe -(CH₂)₃-, n = 1, X et D représentent le groupe 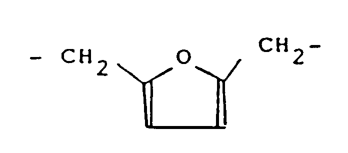 et R₁ et R₂ représentent le groupe -CH₂-COOH.

20. Procédé selon la revendication 11 ou 12, caractérisé en ce que dans le ligand de formule I A et B représentent le groupe -CH₂-CH₂, n = 1, X et D représentent le groupe 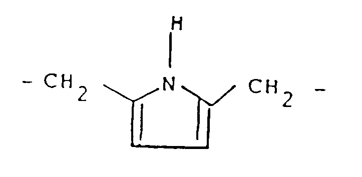 et R₁ et R₂ représentent le groupe -CH₂-COOH.

21. Procédé selon la revendication 11, comprenant un complexe métallique dans lequel l'ion métallique est choisi parmi le gadolinium, l'europium, le dysprosium, le fer (Fe³⁺), le manganèse (Mn²⁺), et le baryum.

22. Procédé selon l'une quelconque des revendications précédentes, dans lequel la composition est constituée par une solution dans un solvant aqueux.

23. Procédé selon l'une quelconque des revendications 11 à 22, dans laquelle le métal est choisi parmi Gd³⁺, Mn²⁺, Fe³⁺, Dy³⁺, Pr³⁺ Eu³⁺, Tb³⁺ et Yb³⁺, pour la préparation d'une composition de diagnostic pour l'imagerie par résonance magnétique.

24. Procédé selon l'une quelconque des revendications 11 à 22 dans lequel le métal est choisi parmi Gd³⁺, Er³⁺, Dy³⁺, Ce³⁺, La³⁺, Bi²⁺ et Ba²⁺ pour la préparation d'une composition de diagnostic pour l'imagerie par rayons X.

## Claims (Claims for the following Contracting State(s): AT, BE, CH, DE, DK, FR, GB, GR, IT, LI, LU, NL, SE)

1. Ligands of formula: 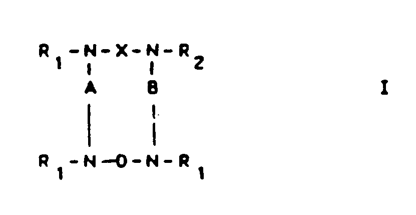 in which
A represents a linear or branched C₂-C₅ alkylene group,
B represents a linear or branched C₂-C₅ alkylene group,
D represents a group X, X being chosen from the groups 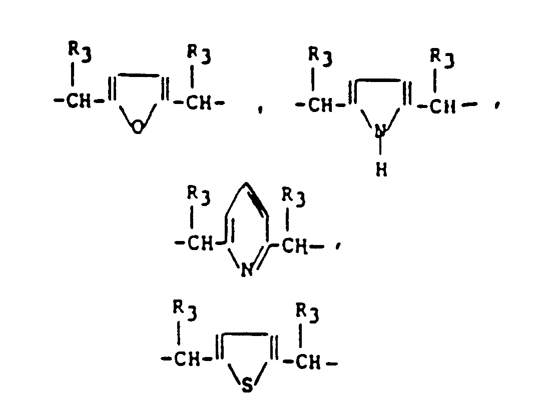 in which R₃ is chosen from a hydrogen atom and a C₁-C₃ alkyl group,
R₁ is chosen from the -CH₂-COOH and -CH₂-PO₃H₂ groups, and
R₂ is chosen from a hydrogen atom, a C₁-C₄ alkyl or C₂-C₄ hydroxyalkyl group and an R₁ group as defined above.

2. Ligands according to Claim 1, characterized in that R₂ is chosen from a hydrogen atom, a C₂-C₄ hydroxyalkyl group and an R₁ group as defined in Claim 1.

3. Ligands according to either of Claims 1 and 2, in which A and B are identical and chosen from ethylene and n-propylene groups, X and D are identical and chosen from the groups 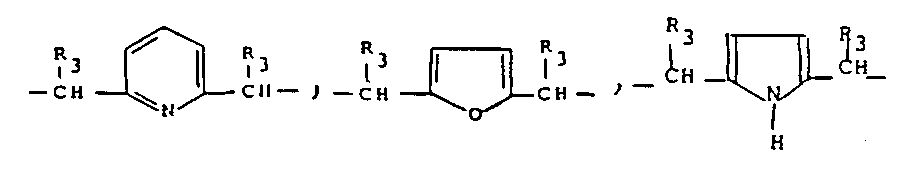 R₃ being chosen from a hydrogen atom and the methyl group, and R₁ and R₂ represent the -CH₂-CO₂H group.

4. Ligand according to Claim 1 or 2, in which A and B represent a -CH₂-CH₂- group, X and D represent the group 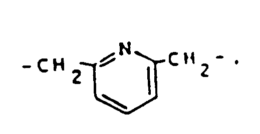 and R₁ and R₂ represent the -CH₂-COOH group.

5. Ligand according to Claim 1 or 2, in which A and B represent the -CH₂-CH₂- group, X and D represent the group 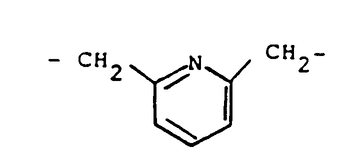 R₁ represents the -CH₂-COOH group and R₂ represents the hydrogen atom.

6. Ligand according to Claim 1 or 2, in which A and B represent the -CH₂-CH₂- group, X and D represent the group 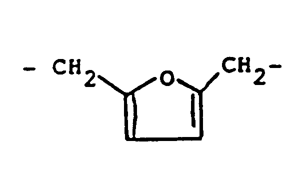 and R₁ and R₂ represent the -CH₂-COOH group.

7. Ligand according to Claim 1 or 2, in which A and B represent the -(CH₂)₃- group, X and D represent the group 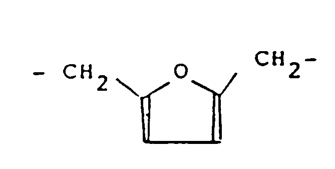 and R₁ and R₂ represent the -CH₂-COOH group.

8. Ligand according to Claim 1 or 2, in which A and B represent the -CH₂-CH₂- group, X and D represent the group 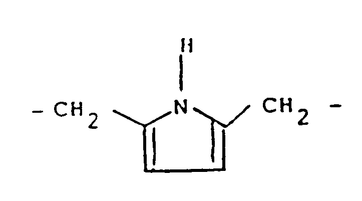 and R₁ and R₂ represent the -CH₂-COOH group.

9. Process for the preparation of a ligand according to either of Claims 1 and 2, characterized in that a cyclic polyamine of formula II 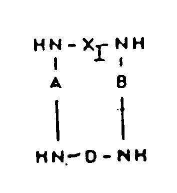 in which A and B have the meaning given in Claim 1 and D represents the group X₁, X₁ being chosen from the groups 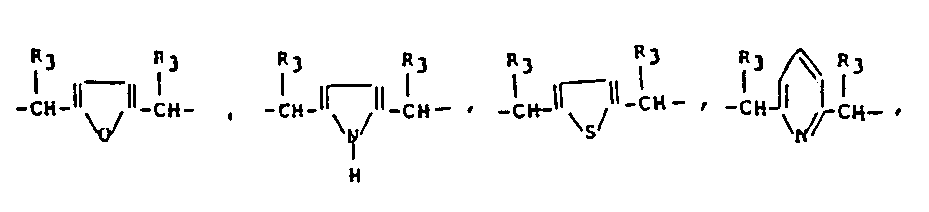 R₃ being as defined in Claim 1,
is reacted with a compound of formula III
Y-CH₂-COOH
in which Y is a labile group, in the presence of NaOH or KOH, when R₁ has the meaning -CH₂-COOH, or with formaldehyde in the presence of phosphonic acid, when R₁ has the meaning -CH₂-PO₃H₂, and optionally a compound of formula IV
Y-R₂
in which Y is a labile group and R₂ is as defined in Claim 1 or Claim 2.

10. Neutral or anionic complexes formed by ligands of formula I according to Claim 1 with metal ions chosen from lanthanide ions, transition metal ions, the barium ion, the bismuth ion and the following radioisotopes:
⁶⁴Cu and ¹⁶⁹Yb,
and the salts of these complexes with pharmaceutically acceptable inorganic or organic bases or basic amino acids.

11. Complexes according to Claim 10, in which the metal ion is chosen from gadolinium, europium, dysprosium, iron (Fe³⁺) manganese (Mn²⁺) and barium.

12. Diagnostic composition which can be administered to man comprising a neutral or anionic complex formed by a ligand of formula: 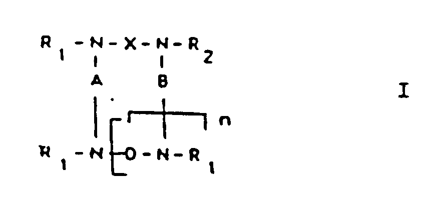 in which
A represents a linear or branched C₂-C₅ alkylene group,
B represents a linear or branched C₂-C₅ alkylene group,
n is equal to 0 or 1,
D is chosen from a linear or branched C₂-C₅ alkylene group and a group X, X being chosen from the groups 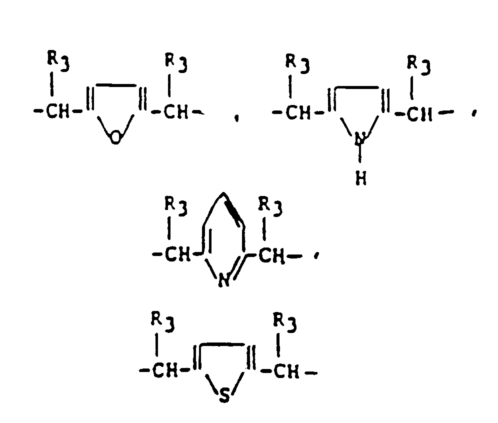 in which R₃ is chosen from a hydrogen atom and a C₁-C₃ alkyl group,
R₁ is chosen from the -CH₂-COOH and -CH₂-PO₃H₂ groups, and
R₂ is chosen from a hydrogen atom, a C₁-C₄ alkyl or C₂-C₄ hydroxyalkyl group and an R₁ group as defined above.

13. Composition according to Claim 12, characterized in that R₂ is chosen from a hydrogen atom, a C₂-C₄ hydroxyalkyl group and an R₁ group as defined in Claim 1.

14. Composition according to Claim 12, characterized in that, in the ligand of formula I, n is equal to 1 and D represents the X group as defined in Claim 1.

15. Composition according to either of Claims 12 and 13, characterized in that, in the ligand of formula I, A and B are identical and chosen from ethylene and n-propylene groups, n is equal to 1, X and D are identical and chosen from the groups 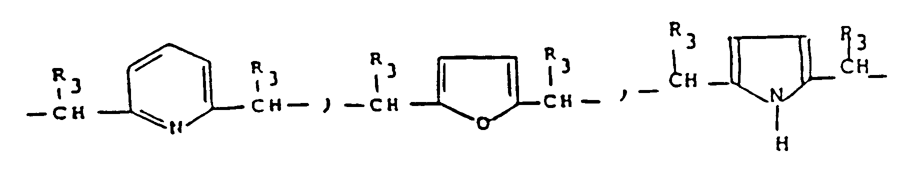 R₃ being chosen from a hydrogen atom and the methyl group, and R₁ and R₂ represent the -CH₂-CO₂H group.

16. Composition according to Claim 12 or 13, characterized in that, in the ligand of formula I, A and B are identical and chosen from ethylene and n-propylene groups, n is equal to 0, X is chosen from the groups 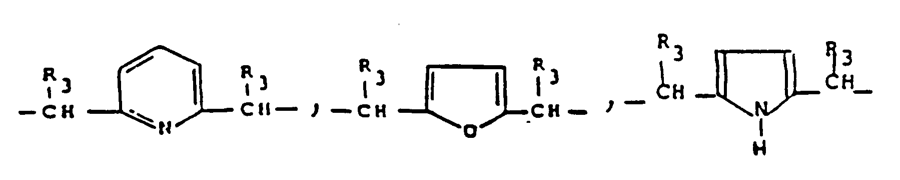 R₃ being chosen from a hydrogen atom and the methyl group, R₁ represents the -CH₂-CO₂H group and R₂ represents a hydrogen atom or the -CH₂-CO₂H group.

17. Composition according to Claim 12 or 13, characterized in that, in the ligand of formula I, A and B represent a -CH₂-CH₂- group, n = 1, X and D represent the group 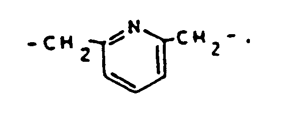 and R₁ and R₂ represent the -CH₂-COOH group.

18. Composition according to Claim 12 or 13, characterized in that, in the ligand of formula I, A and B represent the -CH₂-CH₂- group, n = 1, X and D represent the group 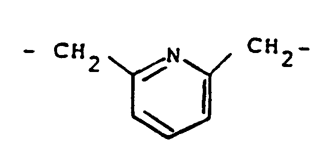 R₁ represents the -CH₂-COOH group and R₂ represents the hydrogen atom.

19. Composition according to Claim 12 or 13, characterized in that, in the ligand of formula I, A and B represent the -CH₂-CH₂- group, n = 1, X and D represent the group 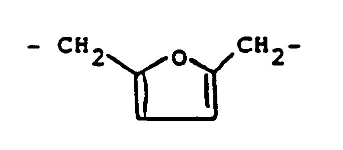 and R₁ and R₂ represent the -CH₂-COOH group.

20. Composition according to Claim 12 or 13, characterized in that, in the ligand of formula I, A and B represent the -(CH₂)₃- group, n = 1, X and D represent the group 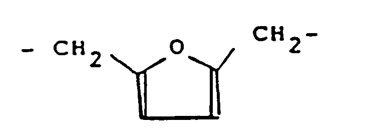 and R₁ and R₂ represent the -CH₂-COOH group.

21. Composition according to Claim 12 or 13, characterized in that, in the ligand of formula I, A and B represent the -CH₂-CH₂- group, n = 1, X and D represent the group 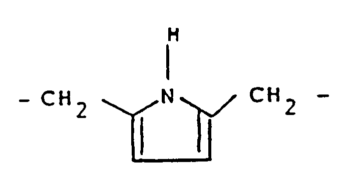 and R₁ and R₂ represent the -CH₂-COOH group.

22. Composition according to Claim 12, comprising a metal complex in which the metal ion is chosen from gadolinium, europium, dysprosium, iron (Fe³⁺), manganese (Mn²⁺) and barium.

23. Composition according to any one of the preceding claims, consisting of a solution in an aqueous solvent.

24. Diagnostic composition according to any one of Claims 12 to 22, in which the metal is chosen from Gd³⁺, Mn²⁺, Fe³⁺, Dy³⁺, Pr³⁺, Eu³⁺, Tb³⁺ and Yb³⁺, for magnetic resonance imaging.

25. Composition according to any one of Claims 12 to 22, in which the metal is chosen from Gd³⁺ Er³⁺, Dy³⁺, Ce³⁺, La³⁺, Bi²⁺ and Ba²⁺, for X-ray imaging.

26. Use of a complex according to either of Claims 10 and 11 for the preparation of a diagnostic composition which can be administered to man intended for magnetic resonance imaging.

27. Use of a complex according to either of Claims 10 and 11 for the preparation of a diagnostic composition which can be administered to man intended for X-ray imaging.

## Claims (Claims for the following Contracting State(s): ES)

1. Process for the preparation of a ligand of formula: 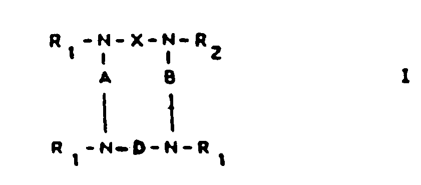 in which
A represents a linear or branched C₂-C₅ alkylene group,
B represents a linear or branched C₂-C₅ alkylene group,
D represents a group X, X being chosen from the groups 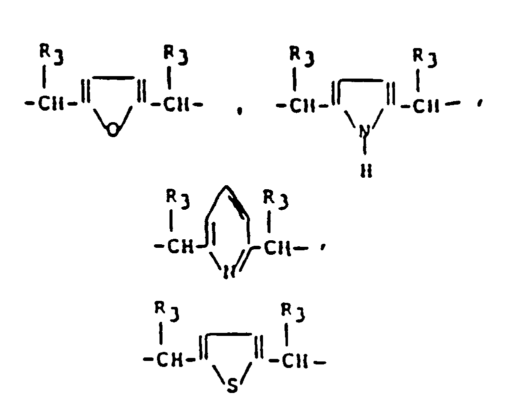 in which R₃ is chosen from a hydrogen atom and a C₁-C₃ alkyl group,
R₁ is chosen from -CH₂-COOH and -CH₂-PO₃H₂ groups, and
R₂ is chosen from a hydrogen atom, a C₁-C₄ alkyl or C₂-C₄ hydroxyalkyl group and an R₁ group as defined above,
characterized in that a cyclic polyamine of formula II 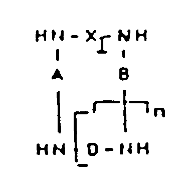 in which A and B have the meaning given above, D represents the group X₁, X₁ being chosen from the groups 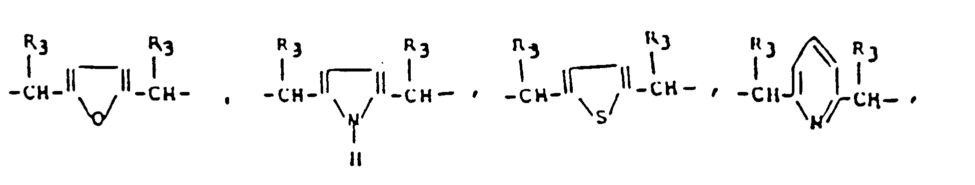 R₃ being as defined above, and n is equal to 1,
is reacted with a compound of formula III
Y-CH₂-COOH
in which Y is a labile group, in the presence of NaOH or KOH, when R₁ has the meaning -CH₂-COOH, or with formaldehyde in the presence of phosphonic acid, when R₁ has the meaning -CH₂-PO₃H₂, and optionally a compound of formula IV
Y-R₂
in which Y is a labile group and R₂ is as defined above.

2. Process according to Claim 1, characterized in that a ligand is prepared in which R₂ is chosen from a hydrogen atom, a C₂-C₄ hydroxyalkyl group and an R₁ group as defined in Claim 1.

3. Process according to Claim 1 or 2, characterized in that a ligand of formula I is prepared in which A and B are identical and chosen from ethylene and n-propylene groups, X and D are identical and chosen from the groups 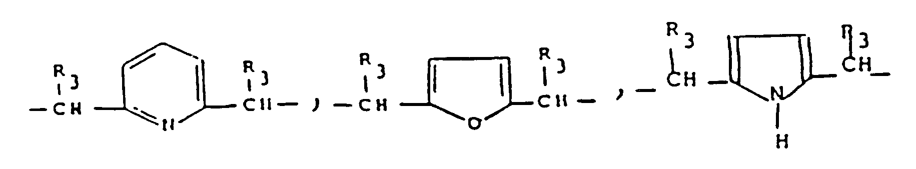 R₃ being chosen from a hydrogen atom and the methyl group, and R₁ and R₂ represent the -CH₂-CO₂H group.

4. Process according to Claim 1 or 2, characterized in that a ligand of formula I is prepared in which A and B represent the -CH₂-CH₂- group, X and D represent the group 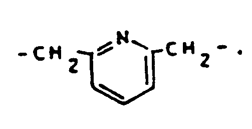 and R₁ and R₂ represent the -CH₂-COOH group.

5. Process according to Claim 1 or 2, characterized in that a ligand of formula I is prepared in which A and B represent the -CH₂-CH₂- group, X and D represent the group 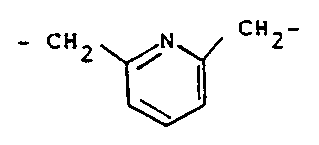 R₁ represents the -CH₂-COOH group and R₂ represents the hydrogen atom.

6. Process according to Claim 1 or 2, characterized in that a ligand of formula I is prepared in which A and B represent the -CH₂-CH₂- group, X and D represent the group 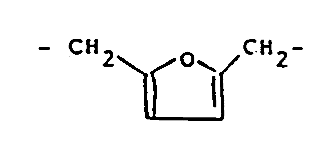 and R₁ and R₂ represent the -CH₂-COOH group.

7. Process according to Claim 1 or 2, characterized in that a ligand of formula I is prepared in which A and B represent the -(CH₂)₃- group, X and D represent the group 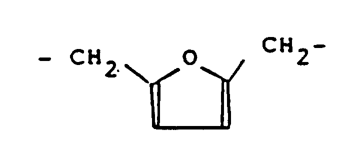 and R₁ and R₂ represent the -CH₂-COOH group.

8. Process according to Claim 1 or 2, characterized in that a ligand of formula I is prepared in which A and B represent the -CH₂-CH₂- group, X and D represent the group 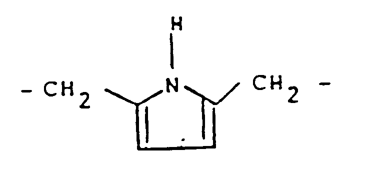 and R₁ and R₂ represent the -CH₂-COOH group.

9. Process for the preparation of neutral or anionic mono- or polymetallic complexes, characterized in that a ligand prepared according to any one of Claims 1 to 8 is reacted with metal ions chosen from lanthanide ions, transition metal ions, the barium ion, the bismuth ion and the following radioisotopes:
⁶⁴Cu and ¹⁶⁹Yb,
and then, optionally, with pharmaceutically acceptable inorganic or organic bases or basic amino acids, in order to obtain the salts of these complexes.

10. Process according to Claim 9, characterized in that metal complexes are prepared in which the metal ion is chosen from gadolinium, europium, dysprosium, iron (Fe³⁺), manganese (Mn²⁺) and barium.

11. Process for the preparation of a diagnostic composition which can be administered to man, characterized in that a neutral or anionic metal complex formed by a ligand of formula 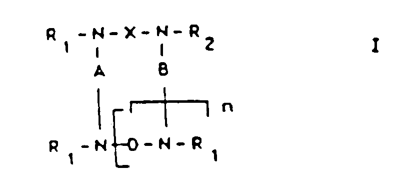 in which
A represents a linear or branched C₂-C₅ alkylene group,
B represents a linear or branched C₂-C₅ alkylene group,
n is equal to 0 or 1,
D is chosen from a linear or branched C₂-C₅ alkylene group and a group X, X being chosen from the groups 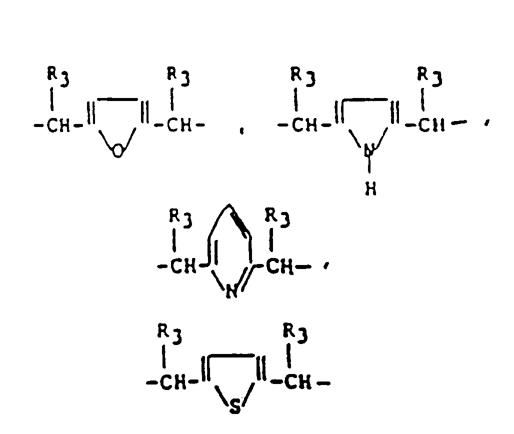 in which R₃ is chosen from a hydrogen atom and a C₁-C₃ alkyl group,
R₁ is chosen from the -CH₂-COOH and -CH₂-PO₃H₂ groups, and
R₂ is chosen from a hydrogen atom, a C₁-C₄ alkyl or C₂-C₄ hydroxyalkyl group and an R₁ group as defined above
or a salt of a complex as defined above with a pharmaceutically acceptable inorganic or organic base or a basic amino acid,
is mixed with a pharmaceutically acceptable excipient.

12. Process according to Claim 11, characterized in that R₂ is chosen from a hydrogen atom, a C₂-C₄ hydroxyalkyl group and an R₁ group as defined in Claim 1.

13. Process according to Claim 11, characterized in that, in the ligand of formula I, n is equal to 1 and D represents the X group as defined in Claim 1.

14. Process according to either of Claims 11 and 12, characterized in that, in the ligand of formula I, A and B are identical and chosen from ethylene and n-propylene groups, n is equal to 1, X and D are identical and chosen from the groups 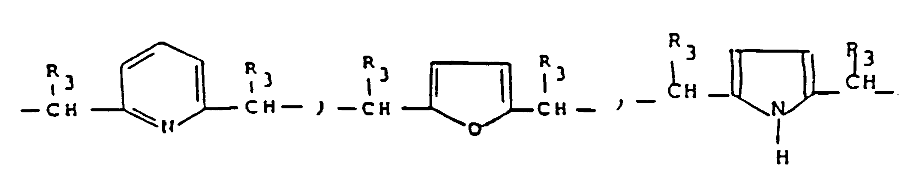 R₃ being chosen from a hydrogen atom and the methyl group, and R₁ and R₂ represent the -CH₂-CO₂H group.

15. Process according to Claim 11 or 12, characterized in that, in the ligand of formula I, A and B are identical and chosen from ethylene and n-propylene groups, n is equal to 0, X is chosen from the groups 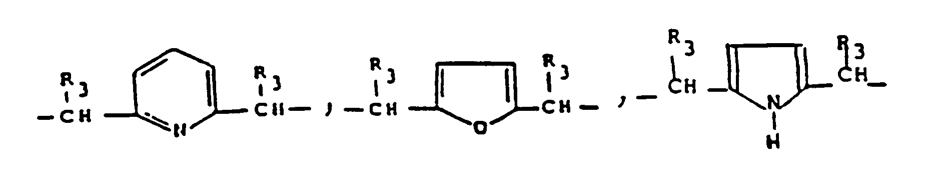 R₃ being chosen from a hydrogen atom and the methyl group, R₁ represents the -CH₂-CO₂H group and R₂ represents a hydrogen atom or the -CH₂-CO₂H group.

16. Process according to Claim 11 or 12, characterized in that, in the ligand of formula I, A and B represent a -CH₂-CH₂- group, n = 1, X and D represent the group 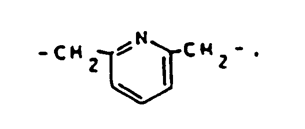 and R₁ and R₂ represent the -CH₂-COOH group.

17. Process according to Claim 11 or 12, characterized in that, in the ligand of formula I, A and B represent the -CH₂-CH₂- group, n = 1, X and D represent the group 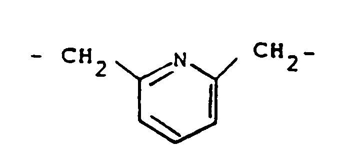 R₁ represents the -CH₂-COOH group and R₂ represents the hydrogen atom.

18. Process according to Claim 11 or 12, characterized in that, in the ligand of formula I, A and B represent the -CH₂-CH₂- group, n = 1, X and D represent the group 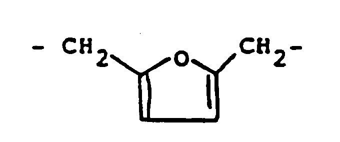 and R₁ and R₂ represent the -CH₂-COOH group.

19. Process according to Claim 11 or 12, characterized in that, in the ligand of formula I, A and B represent the -(CH₂)₃- group, n = 1, X and D represent the group 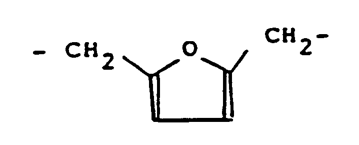 and R₁ and R₂ represent the -CH₂-COOH group.

20. Process according to Claim 11 or 12, characterized in that, in the ligand of formula I, A and B represent the -CH₂-CH₂- group, n = 1, X and D represent the group 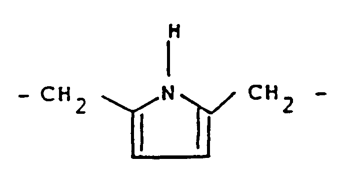 and R₁ and R₂ represent the -CH₂-COOH group.

21. Process according to Claim 11, comprising a metal complex in which the metal ion is chosen from gadolinium, europium, dysprosium, iron (Fe³⁺), manganese (Mn²⁺) and barium.

22. Process according to any one of the preceding claims, in which the composition consists of a solution in an aqueous solvent.

23. Process according to any one of Claims 11 to 22, in which the metal is chosen from Gd³⁺, Mn²⁺, Fe³⁺, Dy³⁺, Pr³⁺, Eu³⁺, Tb³⁺ and Yb³⁺, for the preparation of a diagnostic composition for magnetic resonance imaging.

24. Process according to any one of Claims 11 to 22, in which the metal is chosen from Gd³⁺, Er³⁺, Dy³⁺, Ce³⁺, La³⁺, Bi²⁺ and Ba²⁺, for the preparation of a diagnostic composition for X-ray imaging.

## Patentansprüche (Patentansprüche für folgende(n) Vertragsstaat(en): AT, BE, CH, DE, DK, FR, GB, GR, IT, LI, LU, NL, SE)

1. Liganden mit der Formel: 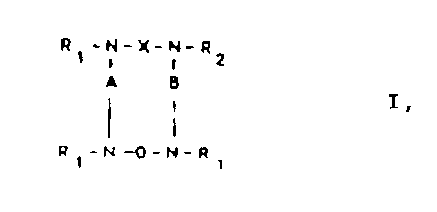 worin
A eine lineare oder verzweigte C₂-C₅-Alkylengruppe darstellt,
B eine lineare oder verzweigte C₂-C₅-Alkylengruppe darstellt,
D eine Gruppe X darstellt, X aus den Gruppen 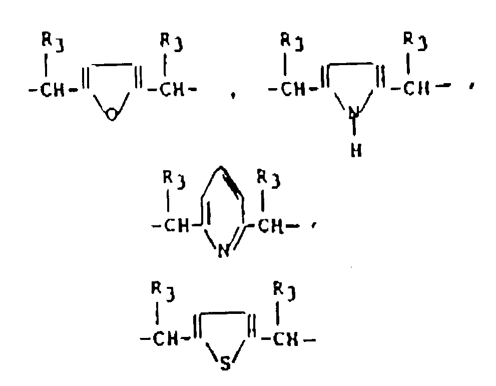 ausgewählt ist, in denen R₃ aus einem Wasserstoffatom und einer C₁-C₃-Gruppe ausgewählt ist,
R₁ aus den Gruppen -CH₂-COOH und -CH₂-PO₃H₂ ausgewählt ist und
R₂ aus einem Wasserstoffatom, einer C₁-C₄-Alkyl-, einer C₂-C₄-Hydroxyalkylgruppe und dem wie zuvor definierten R₁ ausgewählt ist.

2. Liganden nach Anspruch 1, dadurch gekennzeichnet, daß R₂ aus einem Wasserstoffatom, einer C₂-C₄-Hydroxyalkylgruppe und einer wie in Anspruch 1 definierten Gruppe R₁ ausgewählt ist.

3. Liganden nach einem der Ansprüche 1 oder 2, wobei A und B identisch sind und aus der aus Ethylen und n-Propylen bestehenden Gruppe ausgewählt sind, X und D identisch sind und aus den Gruppen 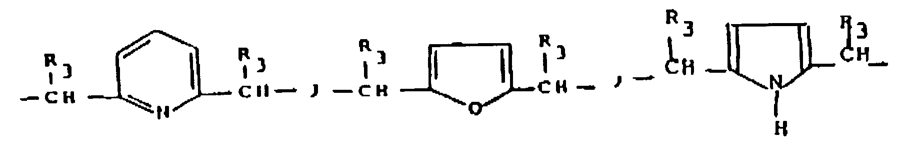 ausgewählt sind,
R₃ aus einem Wasserstoffatom und der Methylgruppe ausgewählt ist und R₁ und R₂ die Gruppe -CH₂-CO₂H darstellen.

4. Ligand nach Anspruch 1 oder 2, wobei A und B die Gruppe -CH₂-CH₂- darstellen, X und D die Gruppe 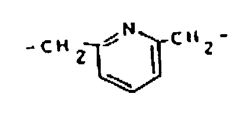 darstellen und R₁ und R₂ die Gruppe -CH₂-COOH darstellen.

5. Ligand nach Anspruch 1 oder 2, wobei A und B die Gruppe -CH₂-CH₂- darstellen, X und D die Gruppe 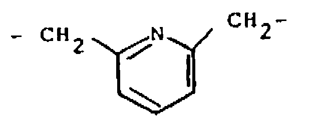 darstellen, R₁ die Gruppe -CH₂-COOH darstellt und R₂ das Wasserstoffatom darstellt.

6. Ligand nach Anspruch 1 oder 2, wobei A und B die Gruppe -CH₂-CH₂- darstellen, X und D die Gruppe 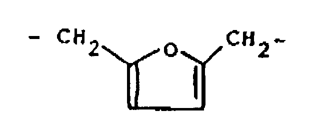 darstellen und R₁ und R₂ die Gruppe -CH₂-COOH darstellen.

7. Ligand nach Anspruch 1 oder 2, wobei A und B die Gruppe -(CH₂)₃- darstellen, X und D die Gruppe 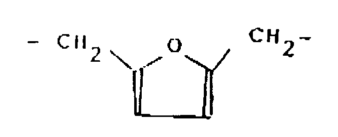 darstellen und R₁ und R₂ die Gruppe -CH₂-COOH darstellen.

8. Ligand nach Anspruch 1 oder 2, wobei A und B die Gruppe -CH₂-CH₂- darstellen, X und D die Gruppe 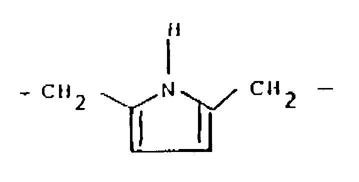 darstellen und R₁ und R₂ die Gruppe -CH₂-COOH darstellen.

9. Verfahren zur Herstellung eines Liganden nach einem der Ansprüche 1 oder 2, dadurch gekennzeichnet, daß man ein cyclisches Polyamin der Formel II 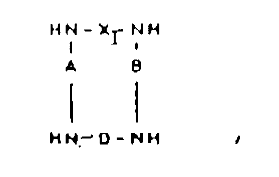 in der A, B die in Anspruch 1 gegebene Bedeutung haben, D die Gruppe X₁ darstellt, X₁ aus den Gruppen 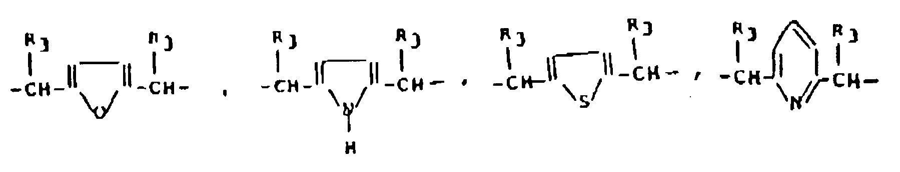 ausgewählt ist, R₃ wie in Anspruch 1 definiert ist,
mit einer Verbindung der Formel III
Y-CH₂-COOH ,
in der Y eine in Gegenwart von NaOH oder KOH labile Gruppe ist, wenn R₁ die Bedeutung -CH₂-COOH hat, oder mit Formaldehyd in Gegenwart von Phosphonsäure, wenn R₁ die Bedeutung -CH₂-PO₃H₂ hat, und gegebenenfalls einer Verbindung der Formel IV
Y-R₂
umsetzt, in der Y eine labile Gruppe ist und R₂ wie in Anspruch 1 oder in Anspruch 2 definiert ist.

10. Neutrale oder anionische Komplexe, gebildet durch Liganden der Formel I nach Anspruch 1, mit Metallionen, ausgewählt aus den Lanthaniden-Ionen, den Übergangsmetall-Ionen, dem Barium-Ion, dem Wismut-Ion und den folgenden Radioisotopen:
⁶⁴Cu und ¹⁶⁹Yb
sowie den Salzen dieser Komplexe mit anorganischen oder pharmazeutisch akzeptablen organischen Basen oder basischen Aminosäuren.

11. Komplexe nach Anspruch 10, in denen das Metallion aus Gadolinium, Europium, Dysprosium, Eisen (Fe³⁺), Mangan (Mn²⁺) und Barium ausgewählt wird.

12. Zusammensetzung zur Diagnose, dem Menschen verabreichbar, umfassend einen neutralen oder anionischen Komplex, gebildet durch einen Liganden mit der Formel: 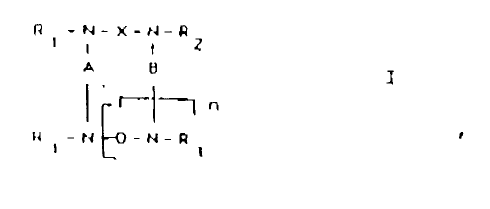 wobei
A eine lineare oder verzweigte C₂-C₅-Alkylengruppe darstellt,
B eine lineare oder verzweigte C₂-C₅-Alkylengruppe darstellt,
n gleich 0 oder 1 ist,
D aus einer linearen oder verzweigten C₂-C₅-Alkylen-Gruppe und einer Gruppe X ausgewählt ist, X aus den Gruppen 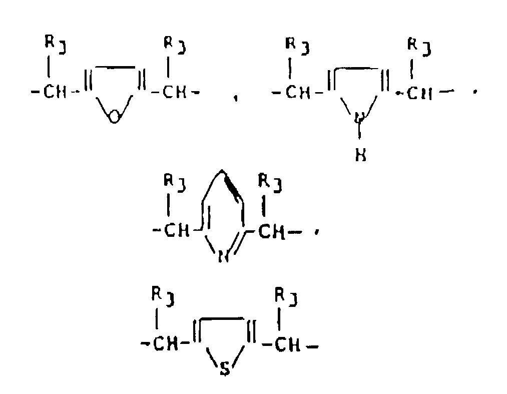 ausgewählt ist, in denen R₃ aus einem Wasserstoffatom und einer C₁-C₃-Alkylgruppe ausgewählt ist,
R₁ aus den Gruppen -CH₂-COOH und -CH₂-PO₃H₂ ausgewählt ist und
R₂ aus einem Wasserstoffatom, einer C₁-C₄-Alkylgruppe, einem C₂-C₄-Hydroxyalkyl und einer wie zuvor definierten Gruppe R₁ ausgewählt ist.

13. Zusammensetzung nach Anspruch 12, dadurch gekennzeichnet, daß R₂ aus einem Wasserstoffatom, einer C₂-C₄-Hydroxy-alkylgruppe und einer wie in Anspruch 1 definierten R₁ ausgewählt ist.

14. Zusammensetzung nach Anspruch 12, dadurch gekennzeichnet, daß im Liganden der Formel I n gleich 1 ist und D die wie in Anspruch 1 definierte Gruppe X darstellt.

15. Zusammensetzung nach einem der Ansprüche 12 oder 13, dadurch gekennzeichnet, daß im Liganden der Formel I A und B identisch sind und aus der aus Ethylen- und n-Propylen bestehenden Gruppe ausgewählt sind, n gleich 1 ist, X und D identisch sind und aus den Gruppen 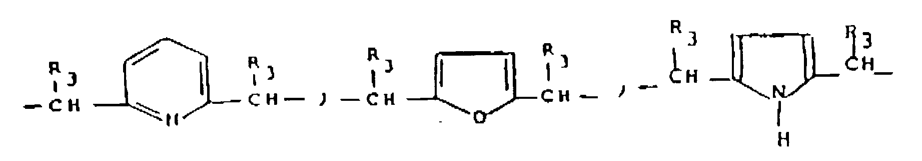 ausgewählt sind, R₃ aus dem Wasserstoffatom und der Methylgruppe ausgewählt ist und R₁ und R₂ die Gruppe -CH₂-CO₂H darstellen.

16. Zusammensetzung nach Anspruch 12 oder 13, dadurch gekennzeichnet, daß im Liganden der Formel I A und B identisch sind und aus der aus Ethylen und n-Propylen bestehenden Gruppe ausgewählt sind, n gleich 0 ist, X aus den Gruppen 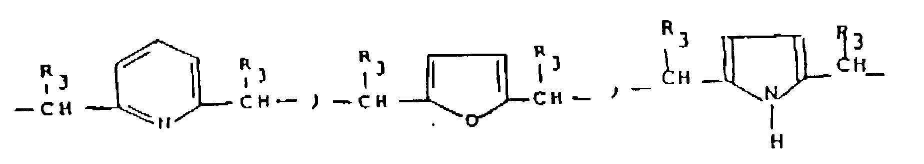 ausgewählt ist, R₃ aus dem Wasserstoffatom und der Methylgruppe ausgewählt ist, R₁ die Gruppe -CH₂-CO₂H darstellt und R₂ ein Wasserstoffatom oder die Gruppe -CH₂-CO₂H darstellt.

17. Zusammensetzung nach Anspruch 12 oder 13, dadurch gekennzeichnet, daß im Liganden der Formel I A und B die Gruppe -CH₂-CH₂- darstellen, n = 1, X und D die Gruppe 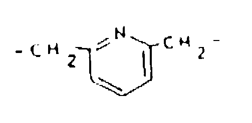 darstellen und R₁ und R₂ die Gruppe -CH₂-COOH darstellen.

18. Zusammensetzung nach Anspruch 12 oder 13, dadurch gekennzeichnet, daß im Liganden der Formel I A und B die Gruppe -CH₂-CH₂- darstellen, n = 1, X und D die Gruppe 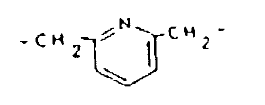 darstellen, R₁ die Gruppe -CH₂-COOH darstellt und R₂ das Wasserstoffatom darstellt.

19. Zusammensetzung nach Anspruch 12 oder 13, dadurch gekennzeichnet, daß im Liganden der Formel I A und B -CH₂-CH₂-darstellen, n = 1, X und D die Gruppe 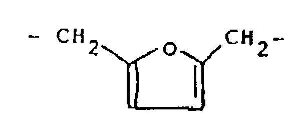 darstellen und R₁ und R₂ die Gruppe -CH₂-COOH darstellen.

20. Zusammensetzung nach Anspruch 12 oder 13, dadurch gekennzeichnet, daß im Liganden der Formel I A und B die Gruppe -(CH₂)₃- darstellen, n = 1, X und D die Gruppe 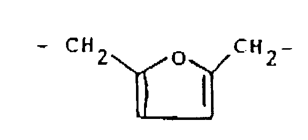 darstellen und R₁ und R₂ die Gruppe -CH₂-COOH darstellen.

21. Zusammensetzung nach Anspruch 12 oder 13, dadurch gekennzeichnet, daß im Liganden der Formel I A und B die Gruppe -CH₂-CH₂- darstellen, n = 1, X und D die Gruppe 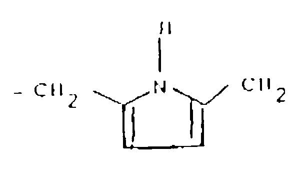 darstellen und R₁ und R₂ die Gruppe -CH₂-COOH darstellen.

22. Zusammensetzung nach Anspruch 12, umfassend einen Metallkomplex, in dem das Metallion aus Gadolinium, Europium, Dysprosium, Eisen (Fe³⁺), Mangan (Mn²⁺) und Barium ausgewählt ist.

23. Zusammensetzung nach einem der vorhergehenden Ansprüche, bestehend aus einer Lösung in einem wässrigen Lösungsmittel.

24. Zusammensetzung zur Diagnose nach einem der Ansprüche 12 bis 22, in dem das Metall aus Gd³⁺, Mn²⁺, Fe³⁺, Dy³⁺, Pr³⁺, Eu³⁺, Tb³⁺ und Yb³⁺ zum Zweck der Bildherstellung durch Kernspinresonanz ausgewählt ist.

25. Zusammensetzung nach einem der Ansprüche 12 bis 22, wobei das Metall aus Gd³⁺, Er³⁺, Dy³⁺, Ce³⁺, La³⁺, Bi²⁺ und Ba²⁺ zum Zweck der Bildherstellung durch Röntgenstrahlen ausgewählt ist.

26. Verwendung eines Komplexes nach einem der Ansprüche 10 oder 11 für die Herstellung einer dem Menschen verabreichbaren Zusammensetzung zur Diagnose, bestimmt zur Bildherstellung durch Kernspinresonanz.

27. Verwendung eines Komplexes nach einem der Ansprüche 10 oder 11 für die Herstellung einer dem Menschen verabreichbaren Zusammensetzung zur Diagnose, bestimmt zur Bildherstellung durch Röntgenstrahlen.

## Patentansprüche (Patentansprüche für folgende(n) Vertragsstaat(en): ES)

1. Verfahren zur Herstellung eines Liganden mit der Formel: 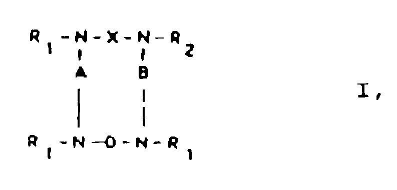 worin
A eine lineare oder verzweigte C₂-C₅-Alkylengruppe darstellt,
B eine lineare oder verzweigte C₂-C₅-Alkylengruppe darstellt,
D eine Gruppe X darstellt, X aus den Gruppen 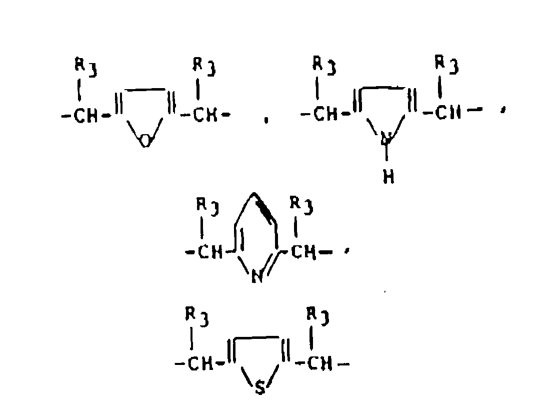 ausgewählt ist, in denen R₃ aus einem Wasserstoffatom und einer C₁-C₃-Gruppe ausgewählt ist,
R₁ aus den Gruppen -CH₂-COOH und -CH₂-PO₃H₂ ausgewählt ist und
R₂ aus einem Wasserstoffatom, einer C₁-C₄-Alkyl-, einer C₂-C₄-Hydroxyalkylgruppe und dem wie zuvor definierten R₁ ausgewählt ist, dadurch gekennzeichnet, daß man ein cyclisches Polyamin der Formel II 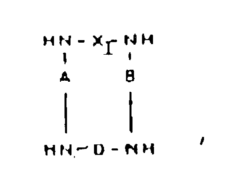 in der A, B die in Anspruch 1 gegebene Bedeutung haben, D die Gruppe X₁ darstellt, X₁ aus den Gruppen 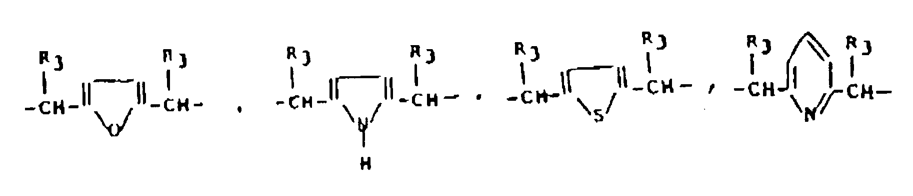 ausgewählt ist, R₃ wie in Anspruch 1 definiert ist, n gleich 1 ist,
mit einer Verbindung der Formel III
Y-CH₂-COOH ,
in der Y eine in Gegenwart von NaOH oder KOH labile Gruppe ist, wenn R₁ die Bedeutung -CH₂-COOH hat, oder mit Formaldehyd in Gegenwart von Phosphonsäure, wenn R₁ die Bedeutung -CH₂-PO₃H₂ hat, und gegebenenfalls einer Verbindung der Formel IV
Y-R₂
umsetzt, in der Y eine labile Gruppe ist und R₂ wie in Anspruch 1 oder in Anspruch 2 definiert ist.

2. Verfahren nach Anspruch 1, dadurch gekennzeichnet, daß man einen Liganden herstellt, in dem R₂ aus einem Wasserstoffatom, einer C₂-C₄-Hydroxyalkylgruppe und einer wie in Anspruch 1 definierten Gruppe R₁ ausgewählt ist.

3. Verfahren nach Anspruch 1 oder 2, dadurch gekennzeichnet, daß man einen Liganden der Formel I herstellt, wobei A und B identisch sind und aus der aus Ethylen und n-Propylen bestehenden Gruppe ausgewählt sind, X und D identisch sind und aus den Gruppen 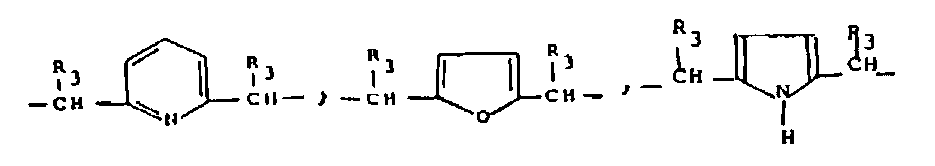 ausgewählt sind,
R₃ aus einem Wasserstoffatom und der Methylgruppe ausgewählt ist und R₁ und R₂ die Gruppe -CH₂-CO₂H darstellen.

4. Verfahren nach Anspruch 1 oder 2, dadurch gekennzeichnet, daß man einen Liganden der Formel I herstellt, wobei A und B die Gruppe -CH₂-CH₂- darstellen, X und D die Gruppe 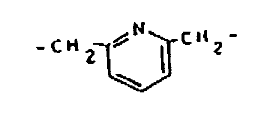 darstellen und R₁ und R₂ die Gruppe -CH₂-COOH darstellen.

5. Verfahren nach Anspruch 1 oder 2, dadurch gekennzeichnet, daß man einen Liganden der Formel I herstellt, wobei A und B die Gruppe -CH₂-CH₂- darstellen, X und D die Gruppe 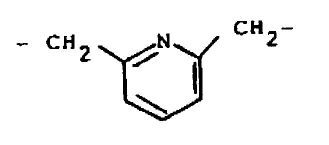 darstellen, R₁ die Gruppe -CH₂-COOH darstellt und R₂ das Wasserstoffatom darstellt.

6. Verfahren nach Anspruch 1 oder 2, dadurch gekennzeichnet, daß man einen Liganden der Formel I herstellt, wobei A und B die Gruppe -CH₂-CH₂- darstellen, X und D die Gruppe 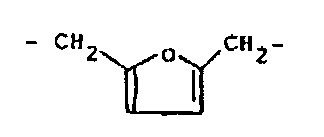 darstellen und R₁ und R₂ die Gruppe -CH₂-COOH darstellen.

7. Verfahren nach Anspruch 1 oder 2, dadurch gekennzeichnet, daß man einen Liganden der Formel I herstellt, wobei A und B die Gruppe -(CH₂)₃- darstellen, X und D die Gruppe 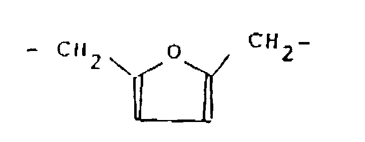 darstellen und R₁ und R₂ die Gruppe -CH₂-COOH darstellen.

8. Verfahren nach Anspruch 1 oder 2, dadurch gekennzeichnet, daß man einen Ligand der Formel I herstellt, wobei A und B die Gruppe -CH₂-CH₂- darstellen, X und D die Gruppe 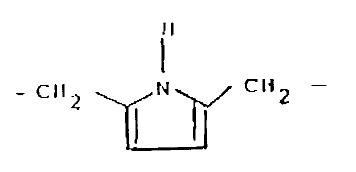 darstellen und R₁ und R₂ die Gruppe -CH₂-COOH darstellen.

9. Verfahren zur Herstellung von monometallischen oder polymetallischen neutralen oder anionischen Komplexen, dadurch gekennzeichnet, daß man einen nach einem der Ansprüche 1 bis 8 hergestellten Liganden mit Metallionen, ausgewählt aus den Lanthaniden-Ionen, den Übergangsmetall-Ionen, dem Barium-Ion, dem Wismut-Ion und den folgenden Radioisotopen:
⁶⁴Cu und ¹⁶⁹Yb,
anschließend gegebenenfalls mit anorganischen oder pharmazeutisch akzeptablen organischen Basen oder basischen Aminosäuren umsetzt, wodurch die Salze dieser Komplexe erhalten werden.

10. Verfahren nach Anspruch 9, dadurch gekennzeichnet, daß man Metallkomplexe herstellt, in denen das Metallion aus Gadolinium, Europium, Dysprosium, Eisen (Fe³⁺), Mangan (Mn²⁺) und Barium ausgewählt wird.

11. Verfahren zur Herstellung einer dem Menschen verabreichbaren Zusammensetzung zur Diagnose, dadurch gekennzeichnet, daß man einen neutralen oder anionischen Komplex, gebildet durch einen Liganden mit der Formel 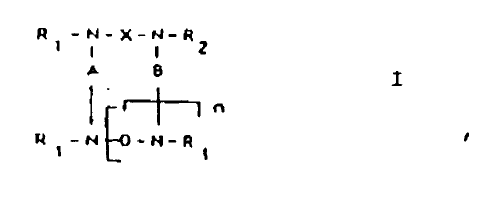 wobei
A eine lineare oder verzweigte C₂-C₅-Alkylengruppe darstellt,
B eine lineare oder verzweigte C₂-C₅-Alkylengruppe darstellt,
n gleich 0 oder 1 ist,
D aus einer linearen oder verzweigten C₂-C₅-Alkylen-Gruppe und einer Gruppe X ausgewählt ist, X aus den Gruppen 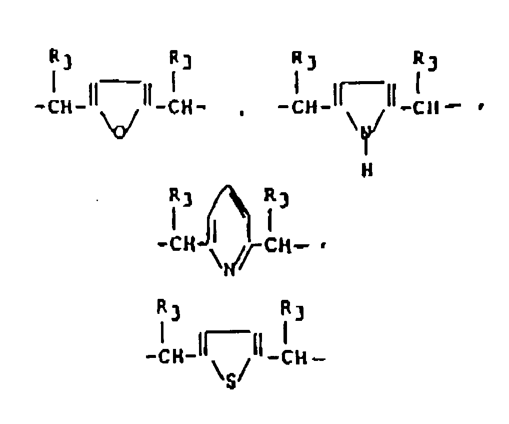 ausgewählt ist, in denen R₃ aus einem Wasserstoffatom und einer C₁-C₃-Alkylgruppe ausgewählt ist,
R₁ aus den Gruppen -CH₂-COOH und -CH₂-PO₃H₂ ausgewählt ist und
R₂ aus einem Wasserstoffatom, einer C₁-C₄-Alkylgruppe, einem C₂-C₄-Hydroxyalkyl und einer wie zuvor definierten Gruppe R₁ ausgewählt ist oder ein Salz eines wie oben definierten Komplexes mit einer anorganischen oder organischen pharmazeutisch akzeptablen Base oder einer basischen Aminosäure mit einer pharmazeutisch akzeptablen Grundmasse vermischt.

12. Verfahren nach Anspruch 11, dadurch gekennzeichnet, daß R₂ aus einem Wasserstoffatom, einer C₂-C₄-Hydroxyalkylgruppe und einer wie in Anspruch 1 definierten Gruppe R₁ ausgewählt ist.

13. Verfahren nach Anspruch 11, dadurch gekennzeichnet, daß im Liganden der Formel I n gleich 1 ist und D die wie in Anspruch 1 definierte Gruppe X darstellt.

14. Verfahren nach einem der Ansprüche 11 oder 12 , dadurch gekennzeichnet, daß im Liganden der Formel I A und B identisch sind und aus der aus Ethylen- und n-Propylen bestehenden Gruppe ausgewählt sind, n gleich 1 ist, X und D identisch sind und aus den Gruppen 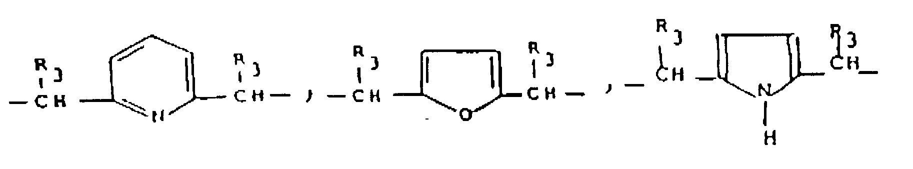 ausgewählt sind, R₃ aus dem Wasserstoffatom und der Methylgruppe ausgewählt ist und R₁ und R₂ die Gruppe -CH₂-CO₂H darstellen.

15. Verfahren nach Anspruch 11 oder 12, dadurch gekennzeichnet, daß im Liganden der Formel I A und B identisch sind und aus der aus Ethylen und n-Propylen bestehenden Gruppe ausgewählt sind, n gleich 0 ist, X aus den Gruppen 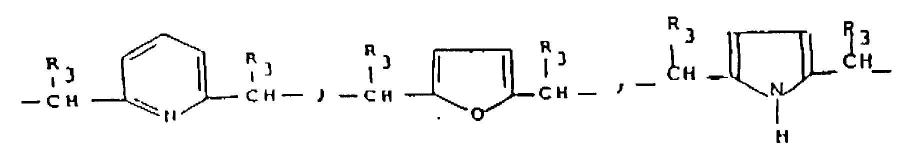 ausgewählt ist, R₃ aus dem Wasserstoffatom und der Methylgruppe ausgewählt ist, R₁ die Gruppe -CH₂-CO₂H darstellt und R₂ ein Wasserstoffatom oder die Gruppe -CH₂-CO₂H darstellt.

16. Verfahren nach Anspruch 11 oder 12, dadurch gekennzeichnet, daß im Liganden der Formel I A und B die Gruppe -CH₂-CH₂- darstellen, n = 1, X und D die Gruppe 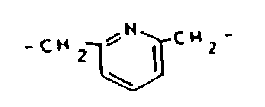 darstellen und R₁ und R₂ die Gruppe -CH₂-COOH darstellen.

17. Verfahren nach Anspruch 11 oder 12 , dadurch gekennzeichnet, daß im Liganden der Formel I A und B die Gruppe -CH₂-CH₂- darstellen, n = 1, X und D die Gruppe 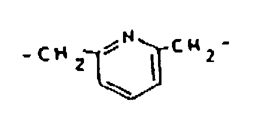 darstellen, R₁ die Gruppe -CH₂-COOH darstellt und R₂ das Wasserstoffatom darstellt.

18. Verfahren nach Anspruch 11 oder 12, dadurch gekennzeichnet, daß im Liganden der Formel I A und B -CH₂-CH₂- darstellen, n = 1, X und D die Gruppe 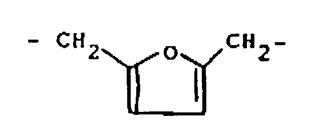 darstellen und R₁ und R₂ die Gruppe -CH₂-COOH darstellen.

19. Verfahren nach Anspruch 11 oder 12, dadurch gekennzeichnet, daß im Liganden der Formel I A und B die Gruppe -(CH₂)₃- darstellen, n = 1, X und D die Gruppe 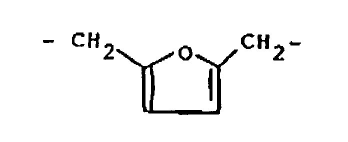 darstellen und R₁ und R₂ die Gruppe -CH₂-COOH darstellen.

20. Verfahren nach Anspruch 11 oder 12 , dadurch gekennzeichnet, daß im Liganden der Formel I A und B die Gruppe -CH₂-CH₂- darstellen, n = 1, X und D die Gruppe 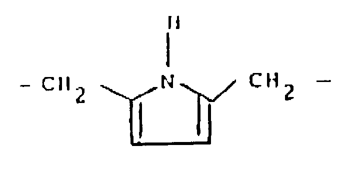 darstellen und R₁ und R₂ die Gruppe -CH₂-COOH darstellen.

21. Verfahren nach Anspruch 11, umfassend einen Metallkomplex, in dem das Metallion aus Gadolinium, Europium, Dysprosium, Eisen (Fe³⁺), Mangan (Mn²⁺) und Barium ausgewählt ist.

22. Verfahren nach einem der vorhergehenden Ansprüche, bestehend aus einer Lösung in einem wässrigen Lösungsmittel.

23. Verfahren nach einem der Ansprüche 11 bis 22, in dem das Metall aus Gd³⁺, Mn²⁺, Fe³⁺, Dy³⁺, Pr³⁺, Eu³⁺, Tb³⁺ und Yb³⁺ zum Zweck der Bildherstellung durch Kernspinresonanz ausgewählt ist.

24. Verfahren nach einem der Ansprüche 11 bis 22, wobei das Metall aus Gd³⁺, Er³⁺, Dy³⁺, Ce³⁺, La³⁺, Bi²⁺ und Ba²⁺ zum Zweck der Bildherstellung durch Röntgenstrahlen ausgewählt ist.
